# FASCICULE DE BREVET EUROPEEN

(11) **EP 2 663 546 B1**
(45) Date de publication et mention de la délivrance du brevet: **09.08.2017**
(21) Numéro de dépôt: 11817338.4
(22) Date de dépôt: 14.12.2011
(51) Int. Cl.: C07C 253/30, C07C 255/19, C07C 227/04, C07C 229/08, C08G 69/04

(54) **PROCEDE DE PRODUCTION DE COMPOSES NITRILE-ACIDE GRAS**
VERFAHREN ZUR HERSTELLUNG VON NITRILFETTSÄUREVERBINDUNGEN
PROCESS FOR PRODUCING NITRILE-FATTY ACID COMPOUNDS

(30) Priorité: 10.01.2011 FR 1150173; 14.06.2011 FR 1155174
(43) Date de publication de la demande: 20.11.2013
(73) Titulaire: Arkema France, 92700 Colombes (FR)
(72) Inventeur: BRANDHORST, Markus, F-69008 Lyon (FR); COUTURIER, Jean-Luc, F-69006 Lyon (FR); DUBOIS, Jean-Luc, F-69390 Millery (FR)
(74) Mandataire: Killis, Andréas
(86) Numéro de dépôt international: PCT/FR2011/052990
(87) Numéro de publication internationale: WO 2012/095575

(56) Documents cités:
- WO-A1-2007/039481
- FR-A1- 2 938 533
- GB-A- 741 739
- GB-A- 743 491
- BIERMANN, URSULA ET AL: "New syntheses with oils and fats as renewable raw materials for the chemical industry", ANGEWANDTE CHEMIE, INTERNATIONAL EDITION, 39(13), 2206 -2224 CODEN: ACIEF5; ISSN: 1433-7851, 2000, XP002648189, cité dans la demande

## Description

L'invention vise un procédé de synthèse d'un nitrile-acide gras, appelé également héminitrile ci-après, à partir d'acides gras insaturés, sous forme acide, ester simple ou « complexe » de type triglycéride, transformé tout d'abord en nitrile gras insaturé qui est soumis à une coupure oxydante en utilisant H₂O₂ comme agent oxydant.

Les nitrile-acides gras de formule générale NC-(CH₂)ₙ-COOH ou de formule NC(CH₂)ₙ(CH=CH)ₘCOOH (formule brute Cₙ₊₂ₘ₊₂H₂ₙ₊₂ₘ₊₁NO₂) dans le cas où la coupure est faite sur un acide polyinsaturé, appelés par la suite héminitriles de diacides ou plus simplement héminitriles, sont des composés intermédiaires utilisables dans la synthèse de toute une gamme de composés « gras » tels que les ω-aminoacides, les α-ω-dinitriles, les α-ω-diamines, α-ω-diacides. On entend par nitrile-acide gras des composés nitrile-acide linéaires ayant de 6 à 15 atomes de carbone.

L'évolution actuelle en matière d'environnement conduit, dans les domaines de l'énergie et de la chimie, à privilégier l'exploitation des matières premières naturelles provenant d'une source renouvelable. C'est la raison pour laquelle d'importants travaux ont été repris pour élaborer sur le plan industriel des procédés utilisant des acides/esters gras (huiles végétales ou graisses animales) comme matière première de fabrication de ces composés gras, par exemple utilisables comme monomères de polymérisation pour obtenir un polyamide.

Il existe une abondante littérature sur la synthèse de divers composés α-ω difonctionnels à partir des acides gras naturels insaturés. Cette littérature est particulièrement focalisée sur l'acide oléique « naturel » pour la fabrication d'ω-amino-acides tels que l'acide 9-amino-nonanoïque qui est le précurseur de la synthèse du Nylon 9. Il faut en effet rappeler que l'industrie des polyamides utilise toute une gamme de monomères constitués par des ω-amino-acides à longue chaîne, usuellement appelés Nylon, caractérisés par la longueur de chaîne méthylène (-CH₂)ₙ séparant deux fonctions amides -CO-NH-. C'est ainsi que sont connus les Nylon-6 (à base d'acide amino-6 héxanoïque), Nylon 7, Nylon 8, Nylon 9, Nylon 11, Nylon 13, et autres.

Les principaux travaux ont porté sur la synthèse de l'acide amino-9-nonanoïque qui est le précurseur du Nylon 9, à partir de l'acide oléique d'origine naturelle.

En ce qui concerne ce monomère particulier, on peut citer l'ouvrage « n-Nylons, Their Synthesis, Structure and Properties » - 1997 Ed.J. Wiley et Sons dont le chapitre 2.9 (pages 381 à 389) est consacré au Nylon 9 (ou 9-Nylon). Cet article fait la synthèse des réalisations et des travaux conduits sur le sujet. On y mentionne, (page 384,) un procédé développé au Japon utilisant l'acide oléïque venant de l'huile de soja comme matière première et consistant à faire une ozonolyse de l'acide oléïque, suivie d'une ammoniation réductrice, conduisant ainsi à l'acide amino-9 nonanoïque.

Pour compléter l'état de l'art relevant de la littérature scientifique, il faut citer les nombreux articles publiés par E. H. Pryde et divers co-auteurs entre 1962 et 1975 dans - Journal of the American Oil Chemists Society - « Amines from Aldehydic derived from the Ozonization of Soybean Esters » Vol. 42, pages 824-827, qui fait référence (page 824,) à des travaux antérieurs réalisés par H. Otsuki et H. Funahashi portant sur l'ozonolyse des acides gras et « Nylon-9 from Unsaturated Fatty Derivatives : Préparation and Characterization », Vol. 52, pages 473-477, dans lequel on compare différentes voies de synthèse de l'acide amino-9 nonanoïque (pages 474 et 475) dont l'une est la voie nitrile, avec formation de l'oléonitrile soumis ensuite à une ozonolyse oxydante.

En ce qui concerne la littérature brevets, on peut citer le brevet GB 741,739 qui décrit la synthèse de l'acide amino-9 nonanoïque à partir d'acides gras insaturés de formule R-CH=CH-(CH₂)₇-COOH avec une première étape d'ammoniation conduisant au nitrile correspondant, soumis en deuxième étape à une ozonolyse oxydante conduisant à l'héminitrile de l'acide azélaïque, transformé en troisième étape par hydrogénation en acide 9-amino-nonanoïque.

La demanderesse a récemment déposé une demande de brevet publiée sous le N° FR 2 938 533 décrivant un procédé de synthèse d'ω-aminoacides gras à partir d'acides/esters gras de formule R₁-CH=CH-(CH₂)ₚ-COOR₂ dans laquelle R₁ est H ou un radical hydrocarboné comprenant de 4 à 11 atomes de carbone et le cas échéant une fonction hydroxyle, R₂ est H ou un radical alkyle comportant de 1 à 4 atomes de carbone et p un indice entier compris entre 2 et 11. Ce procédé comporte deux variantes transitant toutes deux par la formation d'un nitrile ω-insaturé intermédiaire, l'une des variantes comportant une étape d'ammoniation et une étape d'ozonolyse oxydante (du nitrile ω-insaturé).

Par ailleurs, un certain nombre de documents publiés depuis 1990 portent sur la synthèse de diacides (ou diesters) à partir d'acides (ou esters) gras insaturés.

La demande de brevet WO 93/12064 décrit un procédé de synthèse de diacides (ou esters) gras à partir d'acides (ou esters) gras insaturés. Ce procédé utilise l'eau oxygénée comme oxydant pour la coupure oxydante de la double liaison. Il est conduit en une étape et en présence d'un agent de transfert de phase.

Le brevet européen EP 0 666 838 décrit un procédé de synthèse de diacides (ou esters) gras à partir d'acides (ou esters) gras insaturés. Ce procédé est conduit en deux étapes. La première étape utilise l'eau oxygénée pour oxyder la double liaison en formant un diol vicinal. La seconde étape utilise l'oxygène comme oxydant pour obtenir la coupure de la liaison entre les deux atomes de carbone porteurs des fonctions OH.

Cette réaction de coupure de la double liaison des acides gras insaturés a également fait l'objet d'une étude universitaire de E. Santacesaria et al. « Oxidative Cleavage of the Double Bond of Monoenic Fatty Chains in Two Steps : A New Promising Route to Azelaic and Other Industrial Products » publiée dans Ind. Eng. Chem. Res. 2000, 39, 2766-2771, qui analyse les deux étapes de coupure oxydante mentionnées dans le brevet européen EP 0 666 838.

Le point commun à la quasi-totalité des différents schémas décrits est une étape de coupure de la double liaison de l'acide gras insaturé au moyen d'un agent oxydant fort, pour passer d'un acide gras insaturé à longue chaîne à deux molécules saturées grasses à chaîne réduite, l'une α-ω bifonctionnelle et l'autre monofonctionnelle.

La réaction de coupure oxydante de la double liaison qui conduit à la formation de la fonction acide sur les deux carbones de la double liaison est également en elle-même connue. Elle peut être réalisée en utilisant une large gamme d'agents oxydants forts.

Elle peut, par exemple, être réalisée au moyen d'un oxydant fort tel que KMnO₄ sous forme concentrée et à la chaleur. La coupure oxydante peut aussi être obtenue par une voie sulfochromique ou en utilisant le chlorochromate d'ammonium comme oxydant. Angew. Chem. Int. Ed. 2000,39, pp. 2206-2224 décrit la coupure oxydante de la double liaison, soit avec un peracide associé à un catalyseur à base de ruthénium, soit avec H₂O₂ associé avec des catalyseurs à base de Mo, W ou Re.

Cependant, la voie la plus généralement utilisée depuis des décennies est l'ozonolyse. Cette dernière peut être conduite avec l'oléate de méthyle à titre d'exemple, sous forme réductrice selon la réaction suivante :

H₃C-(CH₂)₇-CH=CH-(CH₂)₇-COOCH₃ + (O₃, H₂) → HOC-(CH₂)₇-COOCH₃ + H₃C-(CH₂)₇-COH

ou sous forme oxydante selon la réaction suivante :

H₃C-(CH₂)₇-CH=CH-(CH₂)₇-COOCH₃ + (O₃ + O₂+ H₂O) → H₃C-(CH₂)₇-COOH + COOH-(CH₂)₇-COOCH₃

La fonction introduite sera du type aldéhyde si la coupure est conduite dans des conditions réductrices et du type acide si la coupure est conduite dans des conditions oxydantes.

Le choix entre les deux variantes d'ozonolyse est essentiellement lié au produit final envisagé. Les processus réactionnels avec la formation d'un ozonide et les conditions opératoires de ces réactions ont été largement décrits dans la littérature, notamment dans les articles cités précédemment.

L'utilisation de H₂O₂ comme agent de coupure, déjà mentionnée dans le brevet GB 743,491, a fait l'objet de travaux relativement récents qui ont été mentionnés ci-dessus : WO 93/12064, EP 0 666 838, E. Santacesaria et al, WO 07/039481.

Le problème est de trouver un procédé de synthèse du héminitrile, saturé ou non, plus efficace et/ou moins coûteux que les procédés antérieurs. Or, la demanderesse a découvert que l'utilisation de H₂O₂ comme agent de coupure oxydante dans au moins une des étapes du procédé associé à l'utilisation d'un nitrile insaturé comme réactif permet d'atteindre des performances bien supérieures à celles obtenues avec les procédés de l'art antérieur.

La présente invention a donc pour objet un procédé de synthèse d'un héminitrile de formule CN-(CH₂)ₙ-COOH ou de formule CN-R'-COOH dans lesquelles formules, n est compris entre 4 et 13 y compris les bornes et R' représente un radical alkylène comportant de 4 à 13 atomes de carbone et de 0 à 2 bornes comprises doubles liaisons, avec ladite synthèse étant réalisée à partir d'un composé de type acide y compris ester ou glycéride gras insaturé d'origine naturelle répondant à la formule

(R₁-CH=CH-[(CH₂)_{q}-CH=CH]ₘ-(CH₂)ᵣ-COO-)ₚ-G

dans laquelle formule :
R1 est H, un radical alkyle de 1 à 11 atomes de carbone comportant le cas échéant une fonction hydroxyle,
q est un indice 0 ou 1,
m et p sont des indices entiers, m étant 0, 1 ou 2 et p étant un entier compris entre 1 et 3 y compris les bornes
si p est 1, dans ce cas, G est un H, un radical alkyle de 1 à 11, de préférence de 2 à 11 atomes de carbone ou un radical comportant deux ou trois atomes de carbone, porteur d'une ou deux fonction(s) hydroxyle(s),
si p est 2, dans ce cas, G est le reste d'un diol ou du glycérol porteur d'une fonction hydroxyle,
si p est 2, dans ce cas, G est le reste d'un diol ou du glycérol porteur encore d'une fonction hydroxyle,
si p est 3, dans ce cas, G est le reste du glycérol,
r est un indice entier compris entre 4 et 13 y compris les bornes,
avec les doubles liaisons C=C dans ladite formule pouvant être en conformation cis ou trans
et
avec ledit procédé comportant une première étape d'ammoniation du composé de type acide, ester ou glycéride gras insaturé, conduisant au nitrile insaturé correspondant, lequel nitrile est soumis, dans une deuxième étape, à une coupure oxydante en deux phases successives avec formation de composés intermédiaires de type diols vicinaux, en utilisant H₂O₂ comme agent oxydant, dans au moins une des deux phases, pour conduire audit héminitrile,
ledit procédé mettant en oeuvre en tant qu'acide gras insaturé d'origine naturelle, de l'acide oléïque ou de l'acide lesquérolique ou de l'acide vaccénique ou de l'acide gondoïque et avec comme nitrile utilisé dans la deuxième étape étant l'oléonitrile, le nitrile de l'acide lesquérolique, le nitrile de l'acide vaccénique ou le nitrile de l'acide gondoïque.

Dans la mesure où ledit composé de type acide y compris ester ou glycéride gras insaturé est d'origine naturelle, il peut contenir en faibles quantités d'autres composés, notamment de type acide gras saturé d'origine naturelle, comme c'est le cas dans une huile d'origine naturelle. Par conséquent, la définition du composé de type acide y compris ester ou glycéride gras insaturé signifie également un produit comprenant ledit composé de type acide y compris ester ou glycéride gras insaturé d'origine naturelle de formule précisée ci-dessus. Par exemple, dans le cas de la synthèse, selon la présente l'invention, de l'héminitrile correspondant (acide cyano-8 octanoïque) à partir de l'huile oléique comme matière première d'origine naturelle, cette huile en fonction de sa pureté peut comprendre entre autres et en plus des esters majoritaires purement oléïques y compris triglycérides, des esters minoritaires mixtes d'acide oléïque et d'acide stéarique (acide saturé correspondant à l'olëïque) et palmitique (acide saturé à 16 atomes de carbone). La limitation au composé tel que défini ci-haut (selon formule) comme matière première est un cas particulier de l'invention.

En ce qui concerne la terminologie « compris entre a et b » utilisée ci-haut et pour la suite de la description de l'invention, elle signifie d'une manière générale, sauf indication contraire, l'inclusion des bornes a et b et est à considérer comme étant équivalente à l'expression « allant de a à b » expression qui peut être également utilisée.

Le terme « ester » signifie ester simple, le « glycéride » (mono, di ou tri-) étant considéré comme un ester complexe.

L'héminitrile de formule CN-(CH₂)ₙ-COOH peut être obtenu à partir d'un composé de type acide gras insaturé répondant à la formule R₁-CH=CH-(CH₂)ᵣ-COOG dans laquelle formule, G est un H, un radical alkyle de 1 à 11 et de préférence de 2 à 11 atomes de carbone ou un radical comportant deux ou trois atomes de carbone porteur d'une ou deux fonction(s) hydroxyle(s). L'héminïtrile de formule CN-R'-COOH peut être obtenu à partir d'un composé de type acide gras insaturé répondant à la formule (R₁-CH=CH-[(CH₂)_{q}-CH=CH]ₘ-(CH₂)ᵣ-COO-)ₚ-G, avec R₁, G, m, p, q, r étant définis comme ci-dessus.

Selon un cas particulier du procédé décrit ci-dessus, pour p = 1, G peut être un méthyle, ce qui correspondrait, dans le cas où ledit acide gras insaturé est l'acide oléïque, à un ester oléate de méthyle.

Il est connu, voir notamment la publication de E. Santacesaria citée précédemment, que la réaction de coupure oxydante d'une double liaison est réalisée en deux phases. Au cours de la première phase, on réalise l'oxydation de la double liaison qui conduit à la formation d'un diol vicinal. Dans la deuxième phase, on réalise la rupture de la liaison carbone-carbone entre les deux fonctions hydroxyles avec formation d'un acide saturé ou insaturé d'une part et d'un héminitrile d'autre part, avec l'acide insaturé pouvant être obtenu en particulier dans le cas où la charge comporte des acides gras polyinsaturés. Plus précisément, dans le dernier cas des acides gras polyinsaturés, en fonction de la position de la double liaison objet de la coupure oxydante, on peut obtenir comme produit, soit un acide insaturé, soit un héminitrile insaturé et donc, plus probablement, un mélange des deux. Le schéma réactionnel avec l'oléonitrile à titre d'exemple est le suivant :

CH₃-(CH₂)₇-CH=CH-(CH₂)₇-CN + oxydant → CH₃-(CH₂)₇-CHOH-CHOH-(CH₂)₇-CN

CH₃-(CH₂)₇-CHOH-CHOH-(CH₂)₇-CN + oxydant → CH₃-(CH₂)₇-COOH + COOH-(CH₂)₇-CN

Dans le cas du linoléonitrile (exemple de référence), le schéma réactionnel est :
soit,

   CH₃-(CH₂)₄-CH=CH-CH₂-CH=CH-(CH₂)₇-CN + oxydant → CH₃-(CH₂)₄-CHOH-CHOH- CH₂-CH=CH-(CH₂)₇-CN

   et

   CH₃-(CH₂)₄-CHOH-CHOH- CH₂-CH=CH-(CH₂)₇-CN+ oxydant → CH₃-(CH₂)₄-COOH + HOOC-CH₂-CH=CH-(CH₂)₇-CN

   soit

   CH₃-(CH₂)₄-CH=CH-CH₂-CH=CH-(CH₂)₇-CN + oxydant → CH₃-(CH₂)₄-CH=CH- CH₂-CHOH-CHOH-(CH₂)₇-CN

   et

   CH₃-(CH₂)₄-CH=CH- CH₂-CHOH-CHOH-(CH₂)₇-CN+ oxydant → CH₃-(CH₂)₄-CH=CH-CH₂-COOH + HOOC- CH₂)₇-CN
et le produit final est un mélange des deux héminitriles. Lorsque la charge comporte des acides polyinsaturés, c'est-à-dire quand m est non nul (quand m est égal à 1 ou 2) et l'un des héminitriles répond à la formule générale citée plus haut et avec n = r + (q+2)*m, dans ce cas, on doit au cours d'une étape ultérieure ou antérieure procéder à une hydrogénation de(s) double(s) liaison(s) C=C. Cette hydrogénation pourra se faire notamment après la formation d'un diol vicinal de façon à forcer la coupure oxydante à se faire en une seule position ou en même temps que l'hydrogénation de la fonction nitrile en fonction amine.

Dans cette variante du procédé, avec des nitriles polyinsaturés, la première phase d'oxydation peut aussi conduire à la formation partielle de deux diols vicinaux, ce qui peut entraîner à terme la formation de sous-produits tels que des diacides courts.

Selon des modes variés de réalisation de l'invention, on peut avoir les cas suivants :
- l'héminitrile de formule CN-(CH₂)ₙ-COOH est obtenu à partir d'un composé de type acide gras insaturé répondant à la formule R₁-CH=CH-(CH₂)ᵣ-COOG dans laquelle G est un H, un radical alkyle de 1 à 11 et de préférence de 2 à 11 atomes de carbone ou un radical comportant deux ou trois atomes de carbone et porteur d'une ou deux fonction(s) hydroxyle(s).
- l'héminitrile de formule CN-R'-COOH est obtenu à partir d'un composé de type acide gras insaturé répondant à la formule (R₁-CH=CH-[(CH₂)_{q}-CH=CH]ₘ -(CH₂)ᵣ-COO-)ₚ-G, avec G, R1, m, p, q et r comme définis ci-haut. Le procédé de l'invention peut être conduit selon plusieurs autres variantes.

Selon une première variante, la première phase de la deuxième étape est réalisée en utilisant H₂O₂ comme agent oxydant en présence d'un catalyseur et la deuxième phase de rupture par oxydation (ou rupture oxydante) est réalisée par oxydation par l'oxygène pur ou dilué et/ou par l'air comme agent oxydant (l'oxydant étant l'oxygène moléculaire O₂ dans les deux cas), éventuellement en présence d'un second catalyseur.

Selon une deuxième variante, la première phase est réalisée en utilisant H₂O₂ comme oxydant en présence d'un catalyseur et la deuxième phase de rupture par oxydation est réalisée dans un deuxième réacteur par oxydation au moyen de H₂O₂ comme agent oxydant, éventuellement en présence d'un autre catalyseur.

Selon une troisième variante, les deux phases sont réalisées successivement au sein d'un seul milieu réactionnel et en utilisant H₂O₂ comme oxydant en présence d'un seul catalyseur. WO 93/12064 décrit un procédé utilisant H₂O₂ comme seul oxydant pour synthétiser un diacide à partir d'un acide gras insaturé. Ce procédé nécessite l'utilisation d'un agent de transfert de phase.

Selon une quatrième variante, les deux phases sont réalisées successivement au sein d'un réacteur comportant deux zones : la première zone étant alimentée en H₂O₂ comme agent oxydant et en présence d'un premier catalyseur et la seconde zone avec O₂ (air ou oxygène) comme agent oxydant et en présence d'un second catalyseur, le déplacement du milieu réactionnel d'une zone à l'autre étant assuré par tout moyen convenable.

Ainsi, la première phase de ladite deuxième étape (oxydation) conduisant aux diols vicinaux peut être réalisée par oxydation de(s) double(s) liaison(s) en utilisant H₂O₂ comme agent oxydant, en présence d'un catalyseur d'oxydation.

On entend par composé de type acide gras insaturé naturel un acide ou l'ester (y compris glycéride) gras insaturé correspondant, issu du milieu végétal ou animal, y compris les algues, plus généralement issu du règne végétal et donc renouvelable. Ce composé acide comporte au moins une insaturation oléfinique, localisée en position x par rapport au groupement acide (delta x) et comporte entre 7 et 24 (y compris bornes) atomes de carbone par molécule. Ce composé acide peut être employé après l'hydrolyse des huiles naturelles, mais également directement sous forme de glycérides.

Ces divers composés acides sont issus des huiles végétales extraites de diverses plantes oléagineuses telles que le tournesol, le colza, le ricin, le lesquerella, la cameline, l'olive, le soja, le palmier, les sapindaceae en particulier l'avocat, l'argousier, la coriandre, le céleri, l'aneth, la carotte, le fenouil, la mangue, le Limnanthes Alba (meadowfoam), des micro-algues ou des graisses animales.

La localisation de la double liaison permet de déterminer la formule du héminitrile final et donc on choisira le composé acide en fonction du héminitrile recherché.

Pour obtenir un héminitrile à 9 atomes de carbone, on peut utiliser l'acide oléique (cis-9-octadécénoïque). Pour obtenir un héminitrile à 11 atomes de carbone, on peut utiliser les acides vaccénique (cis-11-octadécénoïque), gondoïque (cis-11-eicosénoïque), lesquérolique (14-hydroxy-cis-11-eicosénoïque), qui peuvent être obtenus à partir de l'huile de Lesquerella (lesquérolique), de l'huile de Camelina sativa (gondoïque), par déshydratation de l'acide 12-hydroxystéarique (12-HSA), lui-même obtenu par hydrogénation de l'acide ricinoléïque (acide vaccénique et son équivalent trans) et de l'acide linoléique conjugué (9-11-octadécadiénoïque), obtenu, par exemple, par déshydratation de l'acide ricinoléique.

Les composés acides les plus importants dans la nature, dans l'ordre d'importance, sont ceux qui donnent les acides, insaturés en C9 (insaturés en position 9), puis en C13 et puis en C11, car ils sont les plus largement disponibles.

Un des acides préférés, avec l'acide gondoïque et lesquérolique, pour obtenir un heminitrile à 11 atomes de carbone, est l'acide vaccénique.

De préférence, l'acide vaccénique est d'origine naturelle, c'est-à-dire issu du milieu végétal ou animal, y compris les algues, plus généralement du règne végétal et donc renouvelable. Ainsi, selon un mode de réalisation préféré, l'invention a pour objet un procédé de synthèse d'un héminitrile à partir d'acide vaccénique d'origine naturelle, en tant que composé de type acide gras insaturé (y compris dérivés esters ou glycérides).

Les voies décrites ci-dessous permettent d'obtenir un acide vaccénique d'origine renouvelable :
- L'acide vaccénique peut être obtenu directement à partir de végétaux, en particulier par extraction, à partir de pulpe de mangue, d'argousier, d'huile d'argousier ou de dérivés d'origine animale tels que le beurre.
- L'acide vaccénique peut également être obtenu en modifiant génétiquement des plantes telles que le carthame, la cameline ou encore *Arabidopsis thaliana* comme cela est décrit par Nguyen et al., dans Plant Physiology, December 2010, Vol. 154, pp 1897-1904.
- L'acide vaccénique peut enfin être obtenu à partir de bactéries ou levures génétiquement modifiées, par exemple *Escherichia Coli,* comme cela est décrit par Mendoza et al, dans Journal of bacteriology September 1982, pp 1608-1611.
- Une dernière voie pour obtenir l'acide vaccénique est la déshydratation ou respectivement l'ammoniation de l'acide 12-hydroxystéarique.

Concernant l'acide gondoïque (acide cis 11-eicosenenoïque), il peut être utilisé, comme l'acide vaccénique et l'acide lesquérolique de manière préférée, pour la préparation d'héminitrile undécanoïque.

De préférence, lorsque l'acide gondoïque est mis en oeuvre, celui-ci est d'origine naturelle, c'est-à-dire d'origine végétale (ce qui comprend les algues) ou animale. Dans un mode de réalisation préféré, l'invention a pour objet un procédé de coupure oxydante d'un nitrile gras insaturé obtenu à partir d'acide gondoïque d'origine naturelle, de sorte à obtenir l'héminitrile correspondant.

Les voies suivantes permettent d'obtenir un acide gondoïque d'origine naturelle :
- L'acide gondoïque (cis-11-eicosenoique) peut être obtenu directement à partir de végétaux, en particulier par extraction, à partir de l'huile de Cameline (*Camelina Sativa*) qui contient plus de 15% d'acide gondoïque, d'huile de Colza riche en acide érucique, de Crambe, de Lunaire qui contiennent en général de 2 à 15% d'acide gondoïque, de *Alyssum Maritimum* (teneur de 41,8% en acide gondoïque), de Selenia Grandis (teneur de 58,5% en acide gondoïque), de *Marshallia caespitosa* (teneur de 43,9% en acide gondoïque).
- L'acide gondoïque peut également être obtenu en modifiant génétiquement des plantes telles que la cameline ou encore *Arabidopsis thaliana.*
- L'acide gondoïque peut être obtenu à partir de bactéries ou levures génétiquement modifiées, par exemple *Escherichia Coli.*
- Une dernière voie pour obtenir l'acide gondoïque est l'hydrolyse de l'huile de Jojoba qui est en fait une cire végétale (appelée huile parce que la cire est liquide à température ambiante). Cette cire végétale (ester gras) renferme environ 35% en poids d'acide gondoïque. Son hydrolyse donne un mélange d'acides gras contenant l'acide gondoïque (environ 70% en poids des acides gras) et des alcools gras à chaîne longue qui sont séparés.

JP9-278706 et JP9-279179 décrivent des procédés d'obtention d'acide gondoïque de pureté élevée à partir de l'hydrolyse de l'huile de Jojoba.

Plus particulièrement, l'acide gondoïque peut être obtenu par les voies suivantes :
1) Comme décrit dans les brevets cités JP9-278706 et JP9-279179, à partir de l'huile de Jojoba. Après hydrolyse de l'huile et une première distillation, il y a une extraction à l'urée et obtention d'une coupe acide riche en acide gondoïque et contenant encore quelques traces d'acide érucique, de préférence sans autre acide insaturé.
2) A partir de l'huile de colza érucique HEAR (High Erucique Acide Rapeseed) contenant par exemple de 5 à 15% d'acide gondoïque. On peut aussi utiliser des huiles de Crambe et de Lunaire, par exemple.
3) A partir d'une variété de colza sélectionnée pour sa plus haute teneur en acide gondoïque, par exemple récoltée avant maturité totale de la plante, car le mécanisme d'élongation des chaines grasses passe par la production intermédiaire de plus fortes concentrations en acide gondoïque dans la plante.
4) A partir de l'huile de Cameline, riche en acide gondoique.
5) A partir d'autres plantes comme Alyssum Maritimum riche en acide gondoïque (teneur de 41,8% en acide gondoïque), Selenia Grandis (teneur de 58,5%), *Marshallia Caespitosa* (teneur de 43,9%).

On peut utiliser toutes les plantes citées ci-dessus et d'autres dans des variétés génétiquement modifiées pour fournir des huiles enrichies en acide gondoïque.

Pour tous les acides gras cités ci-dessus, on peut aussi bien utiliser les acides ayant une conformation cis, qu'une conformation trans.

Parmi les acides (et dérivés) utilisables comme matière première à la synthèse des héminitriles de l'invention, on peut citer pour les héminitriles en C9 l'acide oléïque (ou dérivé ester ou glycéride) et pour les héminitriles en C11, l'acide lesquérolique, l'acide vaccénique et l'acide gondoïque, selon le procédé de l'invention impliquant la coupure oxydante du nitrile insaturé correspondant.

Ainsi, la coupure oxydante de l'oléonitrile conduit à l'héminitrile de l'acide nonanedioïque (acide azélaique) qui peut être utilisé à la préparation de l'acide amino-9 nonanoïque, monomère du polyamide 9 (Nylon 9), par l'hydrogénation de sa fonction nitrile et sa conversion en fonction amine.

La coupure oxydante des nitriles de l'acide lesquérolique, de l'acide vaccénique comme de l'acide gondoïque, conduit dans les trois cas à l'héminitrile de l'acide undécanedioïque, lequel par l'hydrogénation de sa fonction nitrile et sa conversion en fonction amine peut être utilisé à la préparation de l'amino-11 acide undécanoïque, monomère du polyamide 11 (Nylon 11). Le procédé de l'invention met en oeuvre de l'acide oléïque, de l'acide lesquérolique, de l'acide vaccénique ou de l'acide gondoïque d'origine naturelle (source renouvelable), en tant qu'acide gras insaturé (ou dérivé ester ou glycéride) utilisé comme matière première de départ.

Ainsi, selon le procédé de l'invention, le nitrile utilisé dans la deuxième étape est l'oléonitrile, le nitrile de l'acide lesquérolique, le nitrile de l'acide vaccénique ou le nitrile de l'acide gondoïque.

Selon un cas particulier, ledit acide gras insaturé d'origine naturelle impliqué dans le procédé selon l'invention est l'acide oléïque ou un ester ou glycéride correspondant.

Selon un autre cas particulier, ledit acide insaturé d'origine naturelle est l'acide gondoïque (acide cis 11-eicosénoïque) ou ester ou glycéride correspondant.

Ainsi, l'ammoniation de l'acide oléïque (ou ester ou glycéride) conduit selon ce procédé par coupure oxydante à l'héminitrile en C9, lequel peut être utilisé pour préparer l'aminoacide en C9 (acide amino-9 nonanoïque) en rajoutant une étape supplémentaire d'hydrogénation (de la fonction nitrile) audit procédé de l'invention.

De la même manière, les acides lesquérolique (14 hydroxy 11 eicosenoique) vaccénique (cis 11-octadécénoïque) et gondoïque (acide cis 11-eicosénoïque) peuvent conduire à la préparation de l'aminoacide en C11 (acide amino-11 undécanoïque) en passant toujours par la coupure oxydante du nitrile insaturé correspondant auxdits acides gras insaturés, coupure suivie de l'hydrogénation de l'héminitrile correspondant. Selon ce procédé, pour l'héminitrile en C11, les acides vaccénique et gondoïque sont préférés et encore plus préféré est l'acide vaccénique

La synthèse des nitriles à partir des acides en utilisant l'ammoniac est bien connue de l'homme de l'art. On peut faire référence à ce sujet à l'Encyclopédie Kirk-Othmer et au brevet GB 741,739 déjà cité. Le schéma réactionnel peut se résumer de la façon suivante :

R-COOH + NH₃ → [R-COO⁻NH₄⁺] → [R-CONH₂] + H₂O → RCN + H₂O

Ce schéma s'applique tout aussi bien aux acides (esters) gras naturels qu'aux acides gras ω-insaturés.

Le procédé peut être conduit, en batch, en phase liquide ou gazeuse ou en continu en phase gazeuse. La réaction est conduite à température élevée et supérieure à 250°C et en présence d'un catalyseur qui est généralement un oxyde métallique et le plus fréquemment l'oxyde de zinc. L'élimination en continu de l'eau formée, en entraînant de surcroît l'ammoniac qui n'a pas réagi, permet un achèvement rapide de la réaction. L'ammoniation en phase liquide est bien adaptée pour les chaînes grasses longues (comportant au moins 10 atomes de carbone). Cependant, en opérant avec des longueurs de chaîne plus courtes, l'ammoniation en phase gaz peut devenir plus appropriée.

Il est également connu de réaliser l'ammoniation en utilisant comme agent d'ammoniation l'urée ou l'acide cyanurique (voir GB 641,955 déjà cité).

L'étape délicate est la coupure. En effet, le choix de l'oxydant et du dérivé de l'acide gras soumis à cette opération sont essentiels à l'obtention de bons résultats. Le choix d'oxydant se porte sur H₂O₂. C'est un oxydant « vert » et bon marché. Il présente par rapport à l'ozone (O₃), l'oxygène (O₂) et aux autres oxydants forts tels que les permanganates, periodates et autres oxydants forts, de nombreux avantages. Il est facile à manipuler, non toxique, disponible en grande quantité sous forme liquide. Il est plus facile à utiliser dans la réaction car il permet d'utiliser une température de réaction modérée, comparée à O₃ qui nécessite du froid. En outre, on peut opérer à une pression proche de la pression atmosphérique alors que O₂ nécessite un travail sous pression. Sa réaction d'oxydation, comparée à celles utilisant O₂ ou O₃, présente une plus faible exothermicité et permet une bonne dissolution de l'oxydant dans le milieu. En outre, il ne laisse que de l'eau comme co-produit, évitant la présence de résidus difficiles à traiter (osmium toxique ou periodate donnant un composé halogéné ou permanganate ou autre oxydant fort).

La quantité de H₂O₂ introduite dans le milieu réactionnel est un facteur important. Cette quantité est toujours au moins égale à la stoechiométrie de la réaction considérée (c'est-à-dire au moins la quantité stoechiométrique - en H₂O₂). La première phase de l'étape (deuxième) de coupure oxydante, conduisant à la formation du diol vicinal, présente une stoechiométrie de 1 (1/1). La quantité de H₂O₂ injectée est telle que le rapport molaire H₂O₂ / nitrile insaturé est généralement compris entre 1/1 et 4/1 (y compris bornes). Ainsi, pour la première phase de l'étape de coupure oxydante, H₂O₂ peut être injecté dans le milieu à une quantité représentant de 1 à 4 équivalents molaires du nitrile insaturé à oxyder, c'est-à-dire à un rapport molaire H₂O₂ / nitrile insaturé allant de 1 à 4, plus particulièrement sous la forme d'une solution aqueuse ayant une teneur en H₂O₂ comprise entre 30 et 70% (y compris bornes) en poids, de préférence entre 50 et 70% (y compris bornes) en poids et de façon plus préférée entre 60 et 70% (y compris bornes) en poids et de préférence en présence d'un catalyseur constitué de dérivés du tungstène, du molybdène ou du vanadium et, plus particulièrement, choisi parmi l'acide tungstique (H₂WO₄), le sel de sodium de cet acide (Na₂WO₄) associé à H₃PO₄, l'acide molybdique (H₂MoO₄) et son sel de sodium (Na₂MoO₄), des hétéro-polyacides tels que H₃[PMo₁₂O₄₀], H₄[SiMo₁₂O₄₀], H₄[SiW₁₂O₄₀], H₃[PW₁₂O₄₀], (NH₄)₁₀[H₂W₁₂O₄₂], le métavanadate de sodium (Na₃VO₄), le métavanadate d'ammonium ((NH₄)₃VO₄) et leurs sels alcalins.

Lorsque la seconde phase de l'étape de coupure oxydante utilise aussi (comme la première phase) H₂O₂ comme agent oxydant, selon la deuxième et la troisième variante du procédé de l'invention, dans ce cas, la réaction de coupure du diol vicinal formé présente une stoechiométrie de 3 (3/1). La quantité de H₂O₂ injectée sera alors telle que le rapport molaire H₂O₂ / diol vicinal est compris entre 3/1 et 10/1 (y compris bornes). Ainsi, la deuxième phase de coupure par oxydation des diols vicinaux peut être conduite avec H₂O₂ comme oxydant de rupture de la liaison C-C entre les hydroxyles vicinaux, injecté sous la forme d'une solution aqueuse ayant une teneur en H₂O₂ comprise entre 30 et 70% (y compris bornes) en poids (ou massique), de préférence entre 50 et 70% (y compris bornes) en poids et, de façon plus préférée, entre 60 et 70% (y compris bornes) en poids et telle que le rapport molaire H₂O₂ / diol vicinal soit compris entre 3/1 et 10/1 (y compris bornes).

Pour la troisième variante du procédé selon la présente invention où la réaction est conduite dans un seul et même réacteur (« one pot » en anglais), le rapport molaire H₂O₂ / nitrile insaturé est compris entre 4/1 et 15/1 (y compris bornes).

L'eau oxygénée est introduite sous forme d'une solution aqueuse. La concentration de cette solution est également à prendre en considération et elle est comprise entre 30 et 70% (y compris bornes) en poids (massique) et de préférence entre 50 et 70% (y compris bornes) en poids et, de façon plus préférée, entre 60 et 70% (y compris bornes) en poids.

Selon une variante de réalisation avantageuse du procédé de l'invention, pour une phase utilisant H₂O₂ comme oxydant, le catalyseur sera introduit de façon séquentielle lors de la conduite de la phase réactionnelle correspondante. Au lieu d'introduire dans le milieu réactionnel la totalité du catalyseur au début de la réaction, celui-ci sera introduit par quantités réduites au long du processus, H₂O₂ pour sa part étant introduit en continu. Dans une variante particulièrement avantageuse, H₂O₂ et le catalyseur sont introduits de façon séquentielle. Il peut être aussi envisagé d'introduire le catalyseur à très faible dose en continu, comme H₂O₂, en veillant cependant à éviter tout contact préalable entre eux. On peut donc procéder à une injection séquentielle des catalyseurs au long du déroulement du processus réactionnel.

Lorsque le procédé est conduit avec O₂ comme oxydant de rupture de la liaison C-C entre les deux hydroxyles vicinaux dans la deuxième phase de la deuxième étape du procédé de l'invention, dans ce cas la quantité de O₂ introduite sera au moins égale à la stoechiométrie (quantité stoechiométrique) nécessaire de la réaction et, de préférence, avec un rapport molaire O₂ / diol vicinal compris entre 3/2 et 100/1 (y compris bornes). En pratique, on peut travailler avec un très large excès d'air, par exemple par bullage d'air dans le milieu car il n'est pas nécessaire que tout l'oxygène réagisse et travailler ainsi à une basse température pour mieux contrôler la sélectivité.

Les catalyseurs utilisables pour ces deux réactions de coupure oxydantes sont connus de l'homme de l'art d'une manière générale.

Les catalyseurs de la première phase sont de préférence constitués de dérivés du tungstène, du molybdène ou du vanadium. On peut citer comme exemple, l'acide tungstique (H₂WO₄), le sel de sodium de cet acide (Na₂WO₄) associé à H₃PO₄, l'acide molybdique (H₂MoO₄) et son sel de sodium (Na₂MoO₄), des hétéro-polyacides tels que H₃[PMo₁₂O₄₀], H₄[SiMo₁₂O₄₀], H₄[SiW₁₂O₄₀], H₃[PW₁₂O₄₀], (NH₄)₁₀[H₂W₁₂O₄₂], le metavanadate de sodium (Na₃VO₄) ou le metavanadate d'ammonium ((NH₄)₃VO₄). D'une manière générale, les sels alcalins des acides cités ci-dessus conviennent également.

Des catalyseurs de ce type seront utilisés dans la variante du procédé conduite dans un seul réacteur avec H₂O₂ comme oxydant.

Les quantités de catalyseur utilisées dans cette première phase sont généralement comprises entre 0,03 et 2% (y compris bornes) en poids par rapport au poids de nitrile traité et, de préférence, entre 0,5 et 2% (y compris bornes) en poids.

Lors de la deuxième phase de coupure du diol vicinal mettant en oeuvre l'oxygène moléculaire comme agent oxydant, on peut utiliser des catalyseurs à base de cobalt, sous forme d'acétate, comme Co(Ac)₂.4H₂O, chlorure, sulfate de cobalt ou des sels de Cu, Cr, Fe, ou de Mn, ainsi que certains des catalyseurs utilisés lors de la première phase tels que l'acide tungstique (H₂WO₄) et son sel de sodium Na₂WO₄ et des mélanges des métaux comme cités ci-dessus, notamment Co/W.

Ainsi, selon un mode particulier du procédé de l'invention, la deuxième phase de ladite deuxième étape de coupure par oxydation des diols vicinaux peut être conduite avec O₂, comme agent oxydant de rupture de la liaison C-C entre lesdits hydroxyles vicinaux, plus particulièrement en présence d'un catalyseur choisi parmi les sels de cobalt tels que l'acétate (Co(Ac)₂.4H₂O), chlorure et sulfate de cobalt ou les sels de Cu, Cr, Fe, Mn, ainsi que les catalyseurs utilisés lors de la première phase, choisis parmi l'acide tungstique (H₂WO₄) et son sel de sodium Na₂WO₄ et des mélanges Co/W.

Plus particulièrement encore, dans la deuxième phase de la deuxième étape du procédé de l'invention, la réaction peut être conduite avec O₂ comme agent oxydant de rupture de la liaison C-C entre les deux hydroxyles vicinaux et, dans ce cas, la quantité de O₂ introduite sera au moins égale à la stoechiométrie (quantité stoechiométrique) nécessaire à ladite réaction et, de préférence, avec un rapport molaire O₂ / diol vicinal compris entre 3/2 et 100/1 (y compris bornes) et, encore plus particulièrement, avec la réaction étant conduite à une température comprise entre 20 et 80°C (y compris bornes) et de préférence entre 40 et 70°C (y compris bornes) et encore plus particulièrement à une pression comprise entre 1 et 50 bars (y compris bornes), de préférence entre 1 et 20 bars (y compris bornes) et encore plus préférentiellement entre 5 et 20 bars (y compris bornes).

Selon une autre variante particulière du procédé de l'invention, ladite deuxième phase de coupure par oxydation des diols vicinaux est conduite avec H₂O₂ comme agent oxydant de rupture de la liaison C-C entre les hydroxyles vicinaux et de préférence avec ledit H₂O₂ étant injecté sous forme d'une solution aqueuse ayant une teneur en H₂O₂ comprise entre 30 et 70% (y compris bornes) en poids (massique), de préférence entre 50 et 70% (y compris bornes) en poids et de façon plus préférée entre 60 et 70% (y compris bornes) en poids et avec un rapport molaire H₂O₂ / diol vicinal compris entre 3/1 et 10/1 (y compris bornes).

Les quantités de catalyseur utilisées dans cette deuxième phase sont comprises entre 0,1 et 3% (y compris bornes) en moles par rapport au diol traité et de préférence entre 1 et 2% (y compris bornes) en moles.

Plus particulièrement, selon le procédé de l'invention, on procède à une injection séquentielle des catalyseurs au long du déroulement du processus réactionnel.

La deuxième étape du procédé de l'invention est conduite à une température comprise entre 20 et 80°C (y compris bornes) et de préférence entre 40 et 70°C (y compris bornes). La réaction peut être conduite dans une large gamme de pressions, à une pression comprise entre 1 et 50 bars (y compris bornes), de préférence entre 1 et 20 bars (y compris bornes) et de façon plus préférée à une pression sensiblement égale à la pression atmosphérique ou légèrement supérieure à celle-ci et comprise entre 1 et 5 bars (bornes non comprises). Cependant, lorsque la deuxième phase de la coupure oxydante est réalisée avec l'oxygène moléculaire (O₂), on peut utiliser une pression plus élevée que la pression utilisée lors de la première phase et généralement comprise entre 5 et 20 bars (y compris bornes).

Selon une variante de mise en oeuvre du procédé de l'invention, deux réacteurs séparés sont utilisés pour la mise en oeuvre de la deuxième étape, avec un réacteur pour la première phase et un autre pour la deuxième phase. Dans ce cas, il est avantageux d'avoir un recyclage dans le réacteur de première phase des diols formés à l'issue de cette phase. Le taux de recyclage est généralement compris entre 1 et 10% en poids (y compris bornes) par rapport au nitrile de départ entrant en réaction. Ainsi, dans ce cas, deux réacteurs séparés sont utilisés pour la mise en oeuvre de la deuxième étape et l'effluent issu de la première phase (1^{er} réacteur) est soumis à une séparation partielle des fractions aqueuse et organique, permettant ainsi l'élimination partielle de la fraction aqueuse et le recyclage en tête du réacteur (1^{er}) de la première phase, d'une partie de la fraction organique, représentant de 1 à 10% en poids dudit nitrile insaturé.

Selon une autre variante du procédé de l'invention, on utilise un seul réacteur avec H₂O₂ comme unique oxydant pour les deux phases de la deuxième étape et avec le rapport molaire H₂O₂ /nitrile étant compris entre 4/1 et 10/1 (y compris bornes).

L'héminitrile obtenu à l'issue du procédé peut être utilisé comme substrat ou matière première de synthèse des ω-aminoacides. L'héminitrile est soumis à une réaction de réduction à l'hydrogène de la fonction nitrile selon le schéma réactionnel suivant dans le cas du dérivé de l'oléonitrile :

HOOC-(CH₂)₇-CN +2 H₂ → HOOC-(CH₂)₇-CH₂NH₂

Cette réduction de la fonction nitrile en amine primaire et l'obtention des ω-aminoacides(esters) gras, à partir des héminitriles en l'occurrence, est bien connue de l'homme de l'art. L'étape de réduction consiste en une hydrogénation classique. Les catalyseurs utilisables sont nombreux mais préférentiellement on utilisera le Nickel et le Cobalt de Raney. Afin de favoriser la formation de l'amine primaire, on opère avec une pression partielle d'ammoniac.

L'héminitrile obtenu à l'issue du procédé peut être utilisé comme substrat de synthèse pour la synthèse de dinitrile par une réaction consécutive avec de l'ammoniac, selon le schéma réactionnel suivant :

CN-(CH₂)ₙ-COOH + NH₃ → CN-(CH₂)ₙ-CN + 2 H₂O

Cette ammoniation de la fonction acide en nitrile est bien connue de l'homme de l'art : voir à ce sujet GB 741,739 déjà cité. La réaction est conduite à température élevée, de préférence supérieure à 250°C et en présence d'un catalyseur qui est généralement un oxyde métallique et le plus fréquemment l'oxyde de zinc.

Le dinitrile hydrogéné conduit à une diamine qui peut être utilisée dans de nombreuses applications dont la préparation de polyamides, notamment en combinaison avec des diacides.

L'héminitrile obtenu à l'issu du procédé peut être utilisé comme substrat pour la synthèse de diacide par hydrolyse de la fonction nitrile selon le schéma réactionnel suivant :

CN-(CH₂)ₙ-COOH + 2 H₂O **→** HOOC-(CH₂)ₙ-COOH + NH₃

L'hydrolyse se fait généralement dans des conditions acides.

Le diacide peut être utilisé dans de nombreuses applications dont la préparation de polyamides, notamment en combinaison avec des diamines.

Dans la première variante du procédé, la première phase de l'oxydation du nitrile est conduite dans un premier réacteur fonctionnant à une température comprise entre 20 et 70°C (y compris bornes) et une pression comprise entre 1 et 5 bars (y compris bornes), en présence d'un catalyseur constitué par de l'acide tungstique ou un catalyseur équivalent, avec une quantité de H₂O₂ comprise entre 1 et 2 équivalents molaires (c'est-à-dire de 1 à 2 moles H₂O₂ par mole de composé traité), introduite sous forme d'une solution aqueuse de H₂O₂ à une teneur comprise entre 35 et 70% en poids (y compris bornes). Au terme de cette première phase, le cas échéant, après une extraction de l'eau, le milieu réactionnel est transféré dans un deuxième réacteur où il est soumis à une oxydation par O₂ en présence d'un catalyseur à base de cobalt ou un catalyseur équivalent, à une température généralement comprise entre 40 et 70°C (y compris bornes) et à une pression comprise entre 5 et 20 bars (y compris bornes), avec un excès d'oxygène moléculaire.

Lorsque deux réacteurs séparés sont utilisés pour la mise en oeuvre de la deuxième étape, l'effluent issu de la première phase peut être soumis à une séparation partielle des fractions aqueuse et organique, permettant ainsi l'élimination partielle de la fraction aqueuse et le recyclage en tête du réacteur de première phase, d'une partie de la fraction organique représentant de 1 à 10% en poids du nitrile insaturé.

Dans la deuxième variante, la première phase est réalisée comme dans la première variante et la deuxième phase de rupture est réalisée dans un deuxième réacteur par oxydation au moyen de H₂O₂ en présence d'acide tungstique ou d'un autre catalyseur. Au terme de la première phase, le milieu réactionnel peut être soumis à une extraction de la phase aqueuse ainsi qu'à un soutirage partiel de la phase organique pour un recyclage dans le réacteur de première phase. Les conditions de température et de pression lors de la deuxième phase sont généralement plus « douces » que dans la première variante.

Dans la troisième variante, les deux phases sont réalisées successivement au sein d'un seul réacteur, le milieu réactionnel étant oxydé par H₂O₂ en présence d'un seul catalyseur généralement constitué d'acide tungstique ou un catalyseur équivalent. La quantité de H₂O₂ introduite est comprise entre 4 et 10 (y compris bornes) équivalents molaires (c'est-à-dire de 4 à 10 moles de H₂O₂ par mole de nitrile) sous la forme d'une solution à teneur en H₂O₂ comprise entre 35 et 70% en poids (y compris bornes). Ainsi, lorsqu'un seul réacteur est utilisé pour les deux phases avec H₂O₂ comme unique oxydant, le rapport molaire H₂O₂ / nitrile peut être compris entre 4/1 et 10/1 (y compris bornes).

Dans la quatrième variante de procédé, les deux phases sont réalisées successivement au sein d'un réacteur comportant deux zones, la première étant alimentée en H₂O₂ comme oxydant en présence d'un premier catalyseur et la seconde avec O₂ (air) en présence d'un second catalyseur, ledit réacteur étant pourvu de moyens pour déplacer le milieu réactionnel de la zone de première phase à celle de deuxième phase. Le déplacement du milieu réactionnel d'une zone à l'autre peut être réalisé, par exemple, au sein d'un réacteur rotatif de type centrifuge, réacteur en forme de cône ou de disque ou tout autre dispositif permettant de réaliser des couches minces (film mince) de liquide.

On peut citer comme exemple à ce sujet, un « Spinning Disk Reactor » tel que décrit dans la documentation technique de la société Protensive (Protensive Limited, BioScience Centre, International Centre for Life, Times Square, Newcastle upon Tyne, NE1 4EP, United Kingdom), un réacteur de type « Rotating Packed Bed Reactor » (Dow Chemical Company) ou encore des réacteurs tels que ceux commercialisés par la société Myers Vacuum, Inc. qui se présentent sous la forme de cuves maintenues en rotation. A titre d'exemple d'équipements réalisant des couches minces de liquide, on peut citer ceux décrits dans les Techniques de l'Ingénieur, numéro J2360-9 de 1988. Dans toutes ces technologies, la force de gravité est remplacée par la force centrifuge ou par une force mécanique pour maintenir un film mince de liquide et favoriser ainsi le transfert de matière gaz-liquide. Ce type de technologie est particulièrement bien adapté au présent procédé puisque les milieux réactionnels s'avèrent être sensibles (la réaction est sensible), non seulement à la variation de température, mais aussi à la viscosité du milieu (milieux visqueux).

Dans les dispositifs de type centrifuge, la première phase de réaction est assurée au voisinage de l'axe de rotation alimenté en substrat (nitrile gras), catalyseur (acide tungstique) et de H₂O₂ à une quantité représentant entre 1 et 4 (y compris bornes) moles de H₂O₂ par mole de nitrile (équivalents molaires), en solution aqueuse à une teneur comprise entre 35 et 70% en poids (y compris bornes) et la deuxième phase au voisinage de la périphérie du dispositif où l'on injecte O₂ en excès, le catalyseur de deuxième phase étant, quant à lui, toujours introduit dans une zone centrale du dispositif. Le produit final de la réaction est récupéré par débordement à la périphérie. Les réacteurs pouvant convenir assurent tous une faible épaisseur de film liquide, balayé à contre courant ou à cocourant par un flux gazeux contenant de l'oxygène moléculaire.

L'utilisation de ce type de dispositif peut être aussi avantageuse dans la variante du procédé où on conduit la phase d'oxydation à l'oxygène moléculaire dans un réacteur indépendant du réacteur de première phase.

Le procédé de l'invention, en plus de la fabrication dudit héminitrile, peut être utilisé directement ou indirectement pour la préparation d'un ω-aminoacide équivalent (correspondant) audit héminitrile, avec ledit procédé comprenant une étape supplémentaire d'hydrogénation de la fonction nitrile dudit héminitrile en la convertissant en fonction amine correspondante.

Selon cette utilisation, le procédé de l'invention peut conduire à la préparation de l'amino-9 acide nonanoïque à partir de l'oléonitrile ou à l'amino-11 acide undécanoïque à partir des nitriles de l'acide lesquérolique ou de l'acide vaccénique ou de l'acide gondoïque de préférence à partir des nitriles de l'acide vaccénique ou de l'acide gondoïque et plus préférentiellement à partir des nitriles de l'acide vaccénique.

Le procédé de l'invention peut également être utilisé pour la préparation des diamines et/ou de diacides correspondant audit héminitrile de l'invention, ainsi obtenu par ledit procédé, comme déjà décrit ci-haut. Ainsi, lesdites diamines et/ou diacides sont utilisables comme lesdits ω-aminoacides en tant que monomères pour l'obtention de polyamides à partir de matières premières d'origine naturelle et de source renouvelable.

L'utilisation préférée du procédé de préparation d'un héminitrile selon la présente invention concerne la préparation de monomères de polyamides, sélectionnés parmi les ω-aminoacides et/ou les diamines et/ou les diacides équivalents audit héminitrile et/ou concerne également l'obtention de polyamides, par la polymérisation desdits monomères.

Plus particulièrement, ladite utilisation concerne la préparation d'un monomère qui est un ω-aminoacide équivalent audit héminitrile et ledit procédé comprend une étape supplémentaire d'hydrogénation de la fonction nitrile dudit héminitrile et sa conversion en fonction amine correspondante.

Encore plus spécifiquement, cette utilisation conduit à la préparation de l'amino-9 acide nonanoïque à partir de la coupure oxydante de l'oléonitrile ou conduit à l'amino-11 acide undécanoïque à partir de la coupure oxydante des nitriles de l'acide lesquérolique ou de l'acide vaccénique ou de l'acide gondoïque, de préférence à partir de la coupure oxydante des nitriles de l'acide vaccénique ou de l'acide gondoïque et plus préférentiellement à partir de la coupure oxydante des nitriles de l'acide vaccénique.

Enfin, l'invention couvre également un procédé de fabrication d'un polyamide qui comprend l'utilisation du procédé de l'invention pour la préparation d'un héminitrile à partir d'un acide gras insaturé de départ, suivie de l'hydrogénation dudit héminitrile pour obtenir un ω-amino acide correspondant et finalement la polymérisation dudit ω-aminoacide pour obtenir ledit polyamide. De manière particulièrement préférée, ladite fabrication est réalisée à partir d'acides gras de départ correspondants (ou de dérivés esters ou huiles desdits acides gras) d'origine naturelle et de source renouvelable. Plus particulièrement, selon ce procédé, ledit polyamide est de préférence le polyamide 9 et ledit acide gras de départ correspondant est l'acide oléïque où ledit polyamide est un polyamide 11 et ledit acide gras de départ correspondant est l'acide vaccénique ou l'acide lesquérolique ou l'acide gondoïque, de préférence l'acide vaccénique ou l'acide gondoïque et plus préférentiellement l'acide vaccénique.

Le procédé de préparation de l'héminitrile selon l'invention est illustré par les exemples qui suivent.

Les méthodes d'analyse utilisées sont exposées dans la suite ci-après.

La composition de la phase organique est analysée par chromatographie en phase gazeuse (GC) avec un Chromatographe HP 5980.

La teneur en eau oxygénée est analysée par la méthode de dosage par le permanganate CEFIC PEROXYGENS H2O2 AM7157. L'indice d'iode est déterminé selon la norme NF EN 14111.

La viscosité est mesurée à 40°C avec un Viscotester VT550 de Haake avec le dispositif de mesure NV.

### Exemples 1 à 12

Les essais décrits ci-dessous illustrent la réaction d'oxydation de l'oléonitrile (ON) ainsi que, à titre comparatif, celle de l'acide oléique (AO) et de l'oléate de méthyle (OM) au moyen d'eau oxygénée et cela en faisant varier les paramètres de la réaction, c'est-à-dire la teneur en H₂O₂ de la solution injectée, les rapports molaires et débits d'injection, à une température de consigne constante (70°C) et sous pression atmosphérique.

Dans un réacteur double enveloppe de 250 cm³ comportant une agitation mécanique, 100 g de composé gras et 1,1 g d'acide tungstique (H₂WO₄; Merck 98%) sont introduits, puis agités et chauffés à 70°C, température maintenue par circulation d'eau thermostatée. L'eau oxygénée est alors ajoutée à des teneurs pondérales variables en fonction des essais, par une pompe péristaltique à vitesses d'addition variables selon les essais. La réaction est arrêtée après 6 h. La phase aqueuse est séparée pour analyse. La phase organique restante est lavée plusieurs fois à l'eau chaude jusqu'à disparition de l'eau oxygénée dans l'eau de lavage.

Les substrats gras introduits proviennent des sources suivantes :
- oléonitrile (ON): ARKEMA avec C_{16:0} : 3%, C_{18:0} : 9,7%, C_{18:1} : 84,7%, C_{18:2} : 1% (% poids).
- acide oléique (AO) : Oleon Radiacid 0210 (C_{18:1} : 72%, C_{18:2} : 9% poids)
- oléate de méthyle (OM) : Aldrich Grade Technique (C_{18:1} : 70% poids)

Les conditions opératoires et les résultats obtenus sont donnés dans le tableau 1 ci-après dans lequel le « rapport molaire » désigne le rapport molaire H₂O₂ / composé gras et AN désigne la présence (O) ou l'absence (N) d'acide nonanoïque, caractéristique de la coupure de la molécule.

**Tableau 1 : conditions opératoires et résultats**

| Exemple n° | Substrat | [H2O2] (%) | Rapport molaire | Débit g/min | Ind d'iode initial | Ind d'iode final | AN |
|---|---|---|---|---|---|---|---|
| 1 | ON | 50 | 6 | 0,24 | 98 | 4 | O |
| 2 | ON | 50 | 4 | 0,48 | 98 | 30 | O |
| 3 | ON | 50 | 6 | 0,48 | 98 | 41 | O |
| 4 | ON | 35 | 4 | 0,34 | 98 | 37 | N |
| 5 | ON | 50 | 4 | 0,48 | 98 | 32 | O |
| 6 | OM | 35 | 1,8 | 0,128 | 91 | 74 | N |
| 7 | OM | 50 | 4 | 0,48 | 91 | 80 | N |
| 8 | OM | 50 | 4 | 0,48 | 91 | 87 | N |
| 9 | AO | 35 | 1,8 | 0,128 | 86 | 9 | N |
| 10 | AO | 50 | 2,7 | 0,5 | 86 | 9 | O |
| 11 | AO | 35 | 1,8 | 0,256 | 86 | 33 | N |
| 12 | AO | 50 | 6 | 0,48 | 86 | 26 | O |

La réaction d'oxydation de l'oléonitrile permet d'abaisser sensiblement l'indice d'iode du milieu (voir exemple 1) marquant la disparition des doubles liaisons (formation de diols ou coupure). La concentration en H₂O₂ a une influence sur la coupure de la molécule traitée (comparer exemples 1, 2, 3 et 5 vs exemple 4) conduisant à la formation d'héminitrile.

La réaction d'oxydation de l'oléate de méthyle ne conduit qu'à une très faible conversion des doubles liaisons quelles que soient les conditions opératoires. Ce dérivé de l'acide oléique ne convient donc pas à la formation de diacides par coupure oxydante.

La réaction d'oxydation de l'acide oléique permet d'abaisser l'indice d'iode du milieu (exemples 9 à 12) et la formation de diacides, avec une concentration convenable en H₂O₂.

### Exemple 13

L'oxydation de l'oléonitrile est une réaction exothermique qui a une incidence sur la température du milieu réactionnel au cours du temps. Le suivi de cette température, mesurée par thermocouple, permet de mieux appréhender le processus d'oxydation.

L'expérience a été conduite comme suit au moyen du réacteur muni de son agitateur utilisé dans les exemples précédents. Dans une première phase, l'oléonitrile contenant 1% poids de catalyseur H₂WO₄ est monté en température par le bain thermostatique à 70°C, puis on injecte en 40 minutes au moyen d'une pompe péristaltique 2 équivalents molaires (2 moles par mole de nitrile) de H₂O₂ en solution à 50%. Au terme de ces 40 minutes, dans une deuxième phase, on stoppe l'injection durant 60 minutes. La solution est laissée à décanter et la phase aqueuse (avec la majorité de catalyseur) est enlevée. Enfin, on introduit au début de la troisième phase 1% en poids de catalyseur H₂WO₄ et on injecte en 10 minutes au moyen de la pompe péristaltique 4 équivalents molaires (4 moles par mole de nitrile) de H₂O₂ en solution à 50%. Le relevé des températures au sein du milieu réactionnel au long de la réaction est donné dans le tableau 2 ci-dessous.

**Tableau 2 : température**

| Temps (min) | 0 | 3 | 5 | 15 | 25 | 40 | 60 | 100 | 103 | 110 |
|---|---|---|---|---|---|---|---|---|---|---|
| T °C | 65,5 | 64,5 | 74,5 | 74 | 71 | 67,5 | 67 | 66 | 65,5 | 75 |

Cet exemple illustre l'avantage que l'on peut tirer d'une injection séquencée de catalyseur et d'H₂O₂.

### Exemple 14 - comparatif

Dans un réacteur double enveloppe de 250 cm³ comportant une agitation mécanique, sont introduits 110 g d'acide oléïque (pureté 72%, contenant 9% en poids d'acide linoléïque) et 1,1 g d'acide tungstique, puis agités et chauffés à 40°C à la pression atmosphérique. 77 g d'eau oxygénée (49% en poids) sont alors ajoutés par une pompe péristaltique à une vitesse de 0,92 cm³/min. La réaction est arrêtée après 24 h. La phase aqueuse est séparée et analysée. La phase organique est lavée plusieurs fois à l'eau chaude jusqu'à disparition de l'eau oxygénée dans l'eau de lavage.

### Exemple 15 - selon l'invention

Dans un réacteur double enveloppe de 250 cm³ comportant une agitation mécanique, sont introduits 92 g d'oléonitrile (pureté 85%, contenant 10% en poids d'octadécanitrile, le nitrile de l'acide octadécanoïque aussi appelé acide stéarique qui est un composé saturé) et 0,9 g d'acide tungstique, puis agités et chauffés à 40°C à la pression atmosphérique. 77 g d'eau oxygénée (à 49% en poids) sont ajoutés par une pompe péristaltique à une vitesse de 0,95 cm³/min. La réaction est arrêtée après 24 h. La phase aqueuse est séparée et analysée. La phase organique est lavée plusieurs fois à l'eau chaude jusqu'à disparition de l'eau oxygénée dans l'eau de lavage.

Les résultats obtenus d'analyse sont résumés dans les tableaux 3 à 6 ci-dessous.

### Teneurs en H₂O₂

**Tableau 3 : teneur H₂O₂**

| | H₂O₂ % en poids initiale | H₂O₂ % en poids après 24 h |
|---|---|---|
| Acide oléïque | 49 | 45 |
| Oléonitrile | 49 | 32 |

La concentration d'eau oxygénée de la phase aqueuse, initialement de 49% en poids, passe à 45% en poids après 24 h de réaction pour l'acide oléïque et à 32% en poids pour l'oléonitrile. La quantité d'eau oxygénée ayant réagi avec l'oléonitrile est sensiblement plus importante que celle ayant réagi avec l'acide. Cela signifie qu'il y a un plus grand avancement de la réaction d'oxydation du substrat, ce que confirment les résultats des tableaux 4 à 6.

### Indice d'iode :

L'indice d'iode qui mesure la concentration en doubles liaisons est déterminé avant et après la réaction.

**Tableau 4 : indice d'iode**

| | Indice d'iode initial (g I₂/ 100 g de produit) | Indice d'iode après 24 h (g I₂/ 100 g de produit) |
|---|---|---|
| Acide oléïque | 86 | 45 |
| Oléonitrile | 99 | 3 |

On constate une nette diminution du nombre de doubles liaisons dans le cas de l'oléonitrile. La réaction entre l'eau oxygénée et l'oléonitrile, correspondant à la première phase du procédé, progresse nettement plus vite que la réaction entre l'acide oléïque et H₂O₂.

### Analyse par GC (chromatographie en phase gazeuse)

L'analyse par chromatographie en phase gazeuse de la phase organique après réaction est effectuée pour déterminer la quantité d'acide nonanoïque formée qui montre que l'oxydation est arrivée au stade de coupure.

**Tableau 5 : analyse d'acide nonanoïque**

| | Acide nonanoïque (% poids) |
|---|---|
| Acide oléïque | 0,04 |
| Oléonitrile | 0,2 |

La formation de l'acide nonanoïque, produit résultant de la coupure de la chaîne grasse (deuxième phase du procédé), est constatée. On note en comparaison par rapport à l'acide oléïque que la concentration de l'acide nonanoïque résultant de la coupure de l'oléonitrile est 5 fois supérieure par rapport à celle obtenue avec l'acide oléïque.

### Mesure de viscosité

La viscosité η de la phase organique du départ (initiale) ainsi que celle obtenue après 24 h de réaction (viscosité finale) sont mesurées en utilisant respectivement des vitesses de rotation de 245 s⁻¹ et 972 s⁻¹.

**Tableau 6 : viscosité**

| | Vitesse de rotation (s⁻¹) | Viscosité (mPa.s) initiale | Viscosité (mPa.s) finale |
|---|---|---|---|
| Acide oléïque | 245 | 24 | 110 |
| Oléonitrile | 245 | 7 | 82 |
| Acide oléïque | 972 | 19 | 90 |
| Oléonitrile | 972 | 7 | 70 |

Les résultats obtenus montrent que la viscosité augmente au cours de la réaction. On constate cependant que la viscosité de la phase organique obtenue avec l'acide oléïque est supérieure à la viscosité obtenue avec l'oléonitrile, bien que la réaction entre l'eau oxygénée et l'acide oléique soit moins avancée (voir tableaux 4 et 5).

### Exemples 16-20 avec ammoniation de l'acide oléïque et oxydation de l'oléonitrile obtenu

### Ammoniation de l'acide oléïque pour préparer l'oléonitrile

Dans un réacteur en verre préalablement séché de 4 litres, muni d'une agitation mécanique, d'un chauffage électrique, d'un déflegmateur, d'un réfrigérant, d'un piège à carboglace et d'un système d'introduction d'ammoniac, on charge environ 2000 g d'acide gras (oléïque). On ajoute une charge catalytique d'oxyde de zinc (0,0625% du poids d'acide gras). Le milieu réactionnel est mis sous agitation, puis chauffé jusqu'à 200°C. Puis, on introduit l'ammoniac gazeux à raison de 0,417 litres/min.Kg. Le milieu réactionnel est porté à 300°C. L'introduction d'ammoniac se poursuit jusqu'à ce que l'indice d'acidité du milieu réactionnel soit inférieur à 0,1 mg de KOH/g. La durée de la réaction est environ 10 h.

En fin de réaction, on refroidit le milieu réactionnel à 40°C et on vidange le réacteur.

Le produit est purifié par distillation pour obtenir l'oléonitrile, utilisé ci-après.

### Coupure oxydante de l'oléonitrile

Dans un réacteur double enveloppe de 250 ml (100 ml pour n° 20) comportant une agitation mécanique, l'oléonitrile comme préparé ci-dessus et l'acide tungstique (H₂WO₄, Merck 98%) sont introduits, puis agités et chauffés à la température indiquée dans le tableau. La température est maintenue par circulation d'eau thermostatée. L'eau oxygénée est alors ajoutée à diverses teneurs pondérales et à diverses vitesses selon les essais, par une pompe péristaltique. La réaction est arrêtée après le temps indiqué. La phase organique est séparée et lavée plusieurs fois à l'eau chaude jusqu'à disparition de l'eau oxygénée dans l'eau de lavage. Après séchage de la phase organique sous vide, la composition est déterminée par chromatographie en phase gazeuse (GC). Les analyses GC sont réalisées sur un appareil du type HP5890 série Il avec une colonne HP5 et avec détecteur FID.

Pour l'essai de l'exemple 16, l'eau oxygénée est ajoutée de la manière suivante : 48 g de H₂O₂ à 70% en poids dans l'eau sont ajoutés, à débit constant sur une durée d'environ 45 min à 150,4 g d'oléonitrile contenant 1,5 g d'acide tungstique. Après environ 3 h, la phase aqueuse est séparée et de nouveau 48 g de H₂O₂ à 70% en poids dans l'eau sont ajoutés, avec le même débit. Cette étape est encore répétée après 6 h, 21 h, 24 h et après 27 h, pour une durée globale de l'essai de 42 h et avec l'addition globale de 288 g d'H₂O₂ à 70% en poids dans l'eau. A chaque rajout d'eau oxygénée, 1,2 g d'acide tungstique est rajouté. Les exemples 19 et 20 ont été effectués avec le réacteur maintenu sous courant d'azote.

Les conditions des exemples 16 à 20 sont rappelées au tableau 7 ci-dessous.

**Tableau 7 : conditions opératoires des exemples 16 à 20**

| REF Exemple | Composition d'oléonitrile | Poids d'oléonitrile (g) | Poids H₂O₂ à X% dans H₂O (g) | Concentration en X % pds H₂O₂ | T (°C) | Durée totale (h) | Poids H₂WO₄ (g) | Fin d'ajout H₂O₂ (min) |
|---|---|---|---|---|---|---|---|---|
| 16 | 1 | 150,4 | 288 | 70 | 70 | 42 | * | * |
| 17 | 1 | 80,5 | 132 | 50 | 70 | 6 | 0,8 | 90 |
| 18 | 1 | 80,7 | 92 | 70 | 70 | 6 | 0,8 | 82 |
| 19 | 2 | 79,9 | 92 | 70 | 70 | 24 | 8,0 | 131 |
| 20 | 2 | 39,6 | 45 | 70 | 80 | 24 | 4,0 | 121 |

| | | | | | | | | |
|---|---|---|---|---|---|---|---|---|
| * voir texte (mode opératoire ci-haut) | | | | | | | | |

Deux compositions (composition 1 et composition 2) ont été utilisées en ce qui concerne l'acide oléïque et l'oléonitrile correspondant obtenu. Ces compositions sont présentées au tableau 8 ci-dessous.

**Tableau 8 : Compositions 1 et 2 des échantillons de l'acide oléïque et de l'oléonitrile correspondant**

| Composant | % en poids | |
|---|---|---|
| | Composition 1 | Composition 2 |
| C14 :0 | 5,3 | 0,1 |
| C16 :1 | - | 0,2 |
| C16 :0 | 8,2 | 3,5 |
| C18 :1 | 68,6 | 82,7 |
| C18 :0 | 5,5 | 3,5 |
| C18 :2 | 4,8 | 0,5 |
| C20 :1 | 3,8 | 0,3 |
| C20 :0 | 3,8 | 0,2 |

Les résultats de rendement molaire, exprimé en % de moles de produit considéré par rapport au nombre de moles d'oléonitrile engagé au départ, sont présentés au tableau 9 ci-dessous.

**Tableau 9 : résultats de rendement molaire**

| | *Rendement molaire (%) | |
|---|---|---|
| REF Exemple | Acide nonanoïque | Acide 8-cyano-octanoïque |
| 16 | 45,8 | 43,3 |
| 17 | 10,4 | 9,3 |
| 18 | 12,4 | 5,9 |
| 19 | 42,4 | 40,8 |
| 20 | 46,9 | 53,3 |

| | | |
|---|---|---|
| *rendement molaire = moles Produit / moles Oléonitrile engagé au départ | | |

### Exemples 21 et 22 avec l'acide gondoïque

### Matières premières utilisées (acide gondoïque) : A et B

A) on procède comme dans le brevet JP 9-278706 (paragraphes 22 à 28) en utilisant de l'huile de Jojoba pour obtenir une source d'acide gondoïque pure à 99%.
B) on produit un mélange riche en acide gondoïque en partant d'huile de colza érucique. Après hydrolyse de l'huile par saponification, puis acidification, les acides gras sont distillés pour isoler les acides légers riches en acide oléïque d'une part et la fraction riche en acide érucique d'autre part. Ce faisant, une troisième fraction riche en acide gondoïque est produite. Cette fraction contient ainsi 1% d'acide palmitique (C16 :0), 11% d'acide oléïque (C18 :1), 12% d'acide linoléique (C18 :2), 50% d'acide gondoïque (C20 :1), 3% d'acide béhénique (C20 :0), 12% d'acide érucique (C22 :1).

### Ammoniation de l'acide gondoïque pour préparer le nitrile correspondant

Dans un réacteur en verre préalablement séché de 4 litres, muni d'une agitation mécanique, d'un chauffage électrique, d'un déflegmateur, d'un réfrigérant, d'un piège à carboglace et d'un système d'introduction d'ammoniac, on charge environ 2000 g d'acide gras (A ou B). On ajoute une charge catalytique d'oxyde de zinc (0,0625% du poids d'acide gras). Le milieu réactionnel est mis sous agitation, puis chauffé jusqu'à 200°C. Puis, on introduit l'ammoniac gazeux à raison de 0,417 litres/min.Kg. Le milieu réactionnel est porté à 300°C. L'introduction d'ammoniac se poursuit jusqu'à ce que l'indice d'acidité du milieu réactionnel soit inférieur à 0,1 mg de KOH/g. La durée de la réaction est environ 10 h.

En fin de réaction, on refroidit le milieu réactionnel à 40°C et on vidange le réacteur.

Le produit est purifié par distillation pour obtenir le nitrile gondoïque.

Le nitrile obtenu à partir de l'échantillon A conduit à moins de produits lourds que B. La distillation permet d'éliminer les produits lourds qui se forment au cours de la conversion de l'acide en nitrile, mais aussi les amides résiduels formés.

### Coupure oxydante du nitrile de l'acide gondoïque

Dans un réacteur double enveloppe de 250 ml (100 ml pour n° 20) comportant une agitation mécanique, les nitriles résultant des acides A ou B et l'acide tungstique (H₂WO₄, Merck 98%) sont introduits, puis agités et chauffés à la température indiquée dans le tableau. La température est maintenue par circulation d'eau thermostatée. L'eau oxygénée est alors ajoutée à diverses teneurs pondérales et à diverses vitesses selon les essais, par une pompe péristaltique. La réaction est arrêtée après le temps indiqué. La phase organique est séparée et lavée plusieurs fois à l'eau chaude jusqu'à disparition de l'eau oxygénée dans l'eau de lavage. Après séchage de la phase organique sous vide, la composition est déterminée par chromatographie en phase gazeuse (GC). Les analyses GC sont réalisées sur un appareil du type HP5890 série Il avec une colonne HP5 et avec détecteur FID.

Pour l'exemple 21 (nitrile A), l'eau oxygénée est ajoutée de la manière suivante : 15 g de H₂O₂ à 70% en poids dans l'eau sont ajoutés, à débit constant sur une durée d'environ 45 min à 80 g de nitrile A contenant 1,5 g d'acide tungstique. Après environ 3 h, la phase aqueuse est séparée et de nouveau 15 g de H₂O₂ à 70% en poids dans l'eau sont ajoutés, avec le même débit. Cette étape est encore répétée après 6 h, 21 h, 24 h et après 27 h, pour une durée globale de l'essai de 42 h et avec l'addition globale de 90 g d'H₂O₂ à 70% en poids dans l'eau. A chaque rajout d'eau oxygénée, 1,5 g d'acide tungstique est rajouté. Les essais ont été effectués avec le réacteur sous courant d'azote. L'essai de l'exemple 22 avec le nitrile B est réalisé dans les mêmes conditions que l'essai de l'exemple 21 (voir tableau 10 ci-dessous).

**Tableau 10 : conditions des essais 21 et 22**

| REF Exemple | Nitrile gondoïque | Poids de nitriles (g) | Poids H₂O₂ à X% dans H₂O (g) | Concentration X (% pds) H₂O₂ | T (°C) | Durée totale (h) | Poids H₂WO₄ (g) |
|---|---|---|---|---|---|---|---|
| 21 | A | 80 | 90 | 70 | 70 | 42 | 9,0 |
| 22 | B | 80 | 90 | 70 | 70 | 42 | 9,0 |

Les résultats de rendement molaire sont présentés au tableau 11 ci-dessous.

**Tableau 11 : résultas de rendement molaire**

| REF Exemple | Rendement molaire (%) | |
|---|---|---|
| | Acide nonanoïque | Acide 10-cyano-decanoïque |
| 21 (nitrile A) | 38 | 36 |
| 22 (nitrile B) | 35 | 33 |

Le rendement molaire est calculé par rapport au nitrile gondoïque initialement présent pour l'acide 10-cyanodécanoïque et par rapport à l'ensemble des nitriles gras en omega-9 présents dans la charge, pour l'acide nonanoïque.

### Exemple 23, avec hydrogénation de l'héminitrile pour obtenir l'aminoacide correspondant

### Héminitrile : acide 10-cyano décanoïque (ou acide cyano-10 décanoïque)

L'exemple 21 est reproduit en poursuivant l'oxydation par H₂O₂ et en renouvelant la phase aqueuse par de l'eau oxygénée à 70% et le catalyseur toutes les 3 heures pendant une durée de 48 h. A la fin de cette phase, la phase aqueuse est éliminée et le produit récupéré est d'abord distillé sous vide pour éliminer l'acide pélargonique qui s'est formé, puis le produit est recristallisé dans l'acide acétique.

### Hydrogénation de l'héminitrile : obtention de l'acide amino-11 undécanoïque

Un catalyseur Ru/SiC est introduit dans un autoclave en inox d'une capacité de 500 ml équipé d'un agitateur électromagnétique. Une solution contenant 2 g d'acide 10-cyanodécanoïque, obtenu ci-haut conformément à l'invention et de solvant mixte composé de 140 ml d'éthanol et de 140 ml d'ammoniaque à 28% en poids d'ammoniac, est introduite dans l'autoclave. Après avoir purgé le réacteur plusieurs fois avec de l'azote, le réacteur est pressurisé à 35 bars avec de l'hydrogène. Le réacteur est ensuite chauffé à 110°C et l'agitation et la température sont maintenues constantes pendant 1,5 h. La réaction ne consomme alors plus d'hydrogène et l'autoclave redescend en température jusqu'à 70°C, puis la pression est réduite à la pression atmosphérique et un liquide incolore est soutiré. Le solvant est ensuite évaporé sous vide à environ 60°C et des cristaux blancs (1,2 g) d'acide amino-11 undécanoïque sont récupérés.

## Revendications

1. Procédé de synthèse d'un héminitrile de formule CN-(CH₂)ₙ-COOH ou de formule CN-R'-COOH dans lesquelles formules, n est compris entre 4 et 13 y compris bornes et R' représente un radical alkylène comportant de 4 à 13 atomes de carbone et de 0 à 2 doubles liaisons,
avec ladite synthèse étant réalisée, à partir d'un composé de type acide y compris ester ou glycéride gras insaturé d'origine naturelle, répondant à la formule
(R₁-CH=CH-[(CH₂)_{q}-CH=CH]ₘ-(CH₂)ᵣ-COO-)ₚ-G
dans laquelle formule :
R1 est H, un radical alkyle de 1 à 11 atomes de carbone comportant le cas échéant une fonction hydroxyle,
q est un indice étant 0 ou 1,
m et p sont des indices entiers, m étant 0, 1 ou 2 et p étant compris entre 1 et 3 y compris bornes,
si p est 1, dans ce cas G est un H, un radical alkyle de 1 à 11, de préférence de 2 à 11 atomes de carbone ou un radical comportant deux ou trois atomes de carbone porteur d'une ou deux fonction(s) hydroxyle(s),
si p est 2, dans ce cas G est le reste d'un diol ou du glycérol porteur d'une fonction hydroxyle,
si p est 3, dans ce cas, G est le reste du glycérol,
r est un indice entier compris entre 4 et 13 y compris bornes,
avec les doubles liaisons C=C dans ladite formule, pouvant être en conformation cis ou trans et avec ledit procédé, comportant une première étape d'ammoniation du composé de type acide, ester ou glycéride gras insaturé, conduisant au nitrile insaturé correspondant, lequel nitrile est soumis, dans une deuxième étape, à une coupure oxydante en deux phases successives avec formation de composés intermédiaires de type diols vicinaux, en utilisant H₂O₂ comme agent oxydant, dans au moins une des deux phases, pour conduire audit héminitrile ledit procédé mettant en oeuvre en tant qu'acide gras insaturé d'origine naturelle, de l'acide oléïque ou de l'acide lesquérolique ou de l'acide vaccénique ou de l'acide gondoïque et avec comme nitrile utilisé dans la deuxième étape étant l'oléonitrile le nitrile de l'acide lesquérolique ou le nitrile de l'acide vaccénique ou le nitrile de l'acide gondoïque.

2. Procédé selon la revendication 1, **caractérisé en ce que** la première phase de la deuxième étape, conduisant aux diols vicinaux, est réalisée par oxydation de(s) double(s) liaison(s) en utilisant H₂O₂ comme agent oxydant, en présence d'un catalyseur d'oxydation.

3. Procédé selon la revendication 2, **caractérisé en ce que** H₂O₂ est injecté dans le milieu à une quantité représentant de 1 à 4 équivalents molaires, c'est-à-dire de 1 à 4 moles de H₂O₂ par mole de nitrile insaturé à oxyder, plus particulièrement sous la forme d'une solution aqueuse ayant une teneur en H₂O₂ comprise entre 30 et 70% y compris bornes en poids, de préférence entre 50 et 70% y compris bornes en poids et de façon plus préférée entre 60 et 70% y compris bornes en poids et de préférence en présence d'un catalyseur constitué de dérivés du tungstène, du molybdène ou du vanadium, plus particulièrement choisi parmi : l'acide tungstique (H₂WO₄), le sel de sodium de cet acide (Na₂WO₄) associé à H₃PO₄, l'acide molybdique (H₂MoO₄) et son sel de sodium (Na₂MoO₄), des hétéro-polyacides, tels que H₃[PMo₁₂O₄₀], H₄[SiMo₁₂O₄₀], H₄[SiW₁₂O₄₀], H₃[PW₁₂O₄₀], (NH₄)₁₀[H₂W₁₂O₄₂], le métavanadate de sodium (Na₃VO₄), le métavanadate d'ammonium ((NH₄)₃VO₄) et leurs sels alcalins.

4. Procédé selon l'une des revendications 2 et 3, **caractérisé en ce que** la réaction de ladite deuxième étape est conduite à une température comprise entre 20 et 80°C y compris bornes et de préférence entre 40 et 70°C y compris bornes et plus particulièrement à une pression comprise entre 1 et 50 bars y compris bornes, de préférence à une pression comprise entre 1 et 20 bars y compris bornes et de façon plus préférée à une pression sensiblement égale à la pression atmosphérique de 1 bar ou légèrement supérieure à celle-ci et comprise entre 1 et 5 bars bornes non comprises.

5. Procédé selon l'une des revendications 2 à 4, **caractérisé en ce que** la deuxième phase de ladite deuxième étape, de coupure par oxydation des diols, est conduite avec O₂ comme oxydant de rupture de la liaison C-C entre les hydroxyles vicinaux, en présence d'un catalyseur choisi parmi les sels de cobalt, tels que sous forme d'acétate (Co(Ac)₂.4H₂O), chlorure, sulfate ou les sels de Cu, Cr, Fe, Mn, ainsi que les catalyseurs utilisés lors de la première phase, choisis parmi l'acide tungstique (H₂WO₄) et son sel de sodium Na₂WO₄ et des mélanges Co/W.

6. Procédé selon l'une des revendications 2 à 4, **caractérisé en ce que** la deuxième phase de coupure par oxydation des diols vicinaux est conduite avec H₂O₂ comme oxydant de rupture de la liaison C-C entre les hydroxyles vicinaux, de préférence avec ledit H₂O₂ étant injecté sous forme d'une solution aqueuse ayant une teneur en H₂O₂ comprise entre 30 et 70% y compris bornes en poids, de préférence entre 50 et 70% y compris bornes en poids et de façon plus préférée entre 60 et 70% y compris bornes en poids et avec un rapport molaire H₂O₂ / diol vicinal compris entre 3/1 et 10/1 y compris bornes.

7. Procédé selon l'une des revendications 1 à 6, utilisant deux réacteurs séparés pour la mise en oeuvre de la deuxième étape, **caractérisé en ce que** l'effluent issu de la première phase est soumis à une séparation partielle des fractions aqueuse et organique, permettant l'élimination partielle de la fraction aqueuse et le recyclage, en tête du réacteur de première phase, d'une partie de la fraction organique, représentant de 1 à 10% en poids dudit nitrile insaturé.

8. Procédé selon l'une des revendications 1 à 7, **caractérisé en ce qu'**on utilise un seul réacteur, avec H₂O₂ comme unique oxydant pour les deux phases, le rapport molaire H₂O₂ / nitrile étant compris entre 4/1 et 10/1 y compris bornes.

9. Procédé selon l'une des revendications 1 à 8, **caractérisé en ce qu'**on procède à une injection séquentielle des catalyseurs au long du déroulement du processus réactionnel.

10. Utilisation du procédé tel que défini selon l'une des revendications 1 à 9, **caractérisée en ce qu'**elle concerne la préparation de monomères de polyamides, sélectionnés parmi les ω-aminoacides et/ou les diamines et/ou les diacides équivalents audit héminitrile et/ou **en ce que** ladite utilisation concerne l'obtention de polyamides.

11. Procédé de fabrication d'un polyamide, **caractérisé en ce qu'**il comprend l'utilisation du procédé tel que défini selon l'une des revendications 1 à 9 pour la préparation d'un héminitrile à partir d'un acide gras insaturé de départ, suivie de l'hydrogénation dudit héminitrile pour obtenir un ω-aminoacide correspondant et finalement la polymérisation dudit ω-aminoacide pour obtenir ledit polyamide.

12. Procédé selon la revendication 11, **caractérisé en ce que** ladite fabrication est réalisée à partir d'acides gras insaturés de départ correspondants ou de dérivés esters ou huiles desdits acides gras d'origine naturelle et de source renouvelable.

13. Procédé selon la revendication 11 ou 12, **caractérisé en ce que** ledit polyamide est :
- le polyamide 9 et ledit acide gras insaturé de départ correspondant est l'acide oléïque,
- le polyamide 11 et ledit acide gras insaturé de départ correspondant est l'acide vaccénique ou l'acide lesquérolique ou l'acide gondoïque, de préférence l'acide vaccénique ou l'acide gondoïque et plus préférentiellement l'acide vaccénique.

## Patentansprüche

1. Verfahren zur Synthese eines Heminitrils der Formel CN-(CH₂)ₙ-COOH oder der Formel CN-R'-COOH, wobei in den Formeln n zwischen 4 und 13 einschließlich der Grenzen liegt und R' für einen Alkylenrest mit 4 bis 13 Kohlenstoffatomen und 0 bis 2 Doppelbindungen steht,
wobei die Synthese ausgehend von einer Verbindung vom Typ ungesättigte Fettsäure einschließlich Ester oder Glycerid natürlichen Ursprungs, die der Formel
(R₁-CH=CH- [(CH₂) _{q}-CH=CH]ₘ - (CH₂) ᵣ-COO-)ₚ -G
entspricht, durchgeführt wird, wobei in der Formel:
R1 für H oder einen Alkylrest mit 1 bis 11 Kohlenstoffatomen und gegebenenfalls einer Hydroxylfunktion steht,
q für einen Index von 0 oder 1 steht,
m und p für ganzzahlige Indices stehen, wobei m für 0, 1 oder 2 steht und p zwischen 1 und 3 einschließlich der Grenzen liegt,
dann, wenn p für 1 steht, G für ein H, einen Alkylrest mit 1 bis 11 und vorzugsweise 2 bis 11 Kohlenstoffatomen oder einen Rest mit zwei oder drei Kohlenstoffatomen, die eine oder zwei Hydroxylfunktionen tragen, steht,
dann, wenn p für 2 steht, G für den Rest eines Diols oder Glycerins mit einer Hydroxylfunktion steht, dann, wenn p für 3 steht, G für den Glycerinrest steht,
r für einen ganzzahligen Index steht, der zwischen 4 und 13 einschließlich der Grenzen liegt,
wobei die C=C-Doppelbindungen in der Formel in cis-oder trans-Konformation vorliegen können und wobei das Verfahren einen ersten Schritt der Ammonisierung der Verbindung vom Typ ungesättigte Fettsäure, Ester oder Glycerid zu dem entsprechenden ungesättigten Nitril umfasst, wobei das Nitril dann in einem zweiten Schritt einer oxidativen Spaltung in zwei aufeinanderfolgenden Phasen unter Bildung von Zwischenverbindungen vom Typ vicinaler Diole unter Verwendung von H₂O₂ als Oxidationsmittel in mindestens einer der beiden Phasen unterworfen wird, was das Heminitril ergibt, wobei bei dem Verfahren als ungesättigte Fettsäure natürlichen Ursprungs Ölsäure oder Lesquerolsäure oder Vaccensäure oder Gondosäure verwendet wird, und wobei es sich bei dem im zweiten Schritt verwendeten Nitril um Oleonitril, Lesquerolsäurenitril oder Vaccensäurenitril oder Gondosäurenitril handelt.

2. Verfahren nach Anspruch 1, **dadurch gekennzeichnet, dass** die erste Phase des zweiten Schritts, der zu den vicinalen Diolen führt, durch Oxidation der Doppelbindung bzw. Doppelbindungen unter Verwendung von H₂O₂ als Oxidationsmittel in Gegenwart eines Oxidationskatalysators durchgeführt wird.

3. Verfahren nach Anspruch 2, **dadurch gekennzeichnet, dass** das H₂O₂ in einer Menge, die 1 bis 4 Moläquivalente repräsentiert, d.h. 1 bis 4 mol H₂O₂ pro Mol zu oxidierendes ungesättigtes Nitril, spezieller in Form einer wässrigen Lösung mit einem H₂O₂-Gehalt zwischen 30 und 70 Gew.-% einschließlich der Grenzen, vorzugsweise zwischen 50 und 70 Gew.-% einschließlich der Grenzen und weiter bevorzugt zwischen 60 und 70 Gew.-% einschließlich der Grenzen und vorzugsweise in Gegenwart eines Katalysators, der aus Wolfram-, Molybdän- oder Vanadiumderivaten besteht, die spezieller aus Wolframsäure (H₂WO₄), dem Natriumsalz dieser Säure (Na₂WO₄) in Kombination mit H₃PO₄, Molybdänsäure (H₂MoO₄) und deren Natriumsalz (Na₂MoO₄), Heteropolysäuren wie H₃ [PMo₁₂O₄₀], H4 [SiMo₁₂O₄₀], H4 [SiW₁₂O₄₀], H₃ [PW₁₂O₄₀], (NH₄) ₁₀ [H₂W₁₂O₄₂], Natriummetavanadat (Na₃VO₄), Ammoniummetavanadat ((NH₄)₃VO₄) und deren Alkalisalzen ausgewählt sind, in das Medium eingeleitet wird.

4. Verfahren nach einem der Ansprüche 2 und 3, **dadurch gekennzeichnet, dass** die Umsetzung des zweiten Schritts bei einer Temperatur zwischen 20 und 80 °C einschließlich der Grenzen und vorzugsweise zwischen 40 und 70 °C einschließlich der Grenzen und spezieller bei einem Druck zwischen 1 und 50 bar einschließlich der Grenzen, vorzugsweise bei einem Druck zwischen 1 und 20 bar einschließlich der Grenzen und weiter bevorzugt bei einem weitgehend dem Atmosphärendruck von 1 bar entsprechenden Druck oder etwas darüber und zwischen 1 und 5 bar einschließlich der Grenzen durchgeführt wird.

5. Verfahren nach einem der Ansprüche 2 bis 4, **dadurch gekennzeichnet, dass** die zweite Phase des zweiten Schritts, die oxidative Spaltung der Diole, mit O₂ als Oxidationsmittel zum Brechen der C-C-Bindung zwischen den vicinalen Hydroxylgruppen in Gegenwart eines Katalysators, der aus Cobaltsalzen wie in Form von Acetat (Co(Ac)_{2·}4H₂O), Chlorid, Sulfat oder Salzen von Cu, Cr, Fe und Mn sowie den in der ersten Phase verwendeten Katalysatoren, die aus Wolframsäure (H₂WO₄) und dessen Natriumsalz Na₂WO₄ ausgewählt sind, und Co/W-Mischungen ausgewählt ist, durchgeführt wird.

6. Verfahren nach einem der Ansprüche 2 bis 4, **dadurch gekennzeichnet, dass** die zweite Phase der oxidativen Spaltung der vicinalen Diole mit H₂O₂ als Oxidationsmittel zum Brechen der C-C-Bindung zwischen den vicinalen Hydroxylgruppen durchgeführt wird, wobei das H₂O₂ vorzugsweise in Form einer wässrigen Lösung mit einem H₂O₂-Gehalt zwischen 30 und 70 Gew.-% einschließlich der Grenzen, vorzugsweise zwischen 50 und 70 Gew.-% einschließlich der Grenzen und weiter bevorzugt zwischen 60 und 70 Gew.-% einschließlich der Grenzen und mit einem Molverhältnis von H₂O₂ zu vicinalem Diol zwischen 3/1 und 10/1 einschließlich der Grenzen eingeleitet wird.

7. Verfahren nach einem der Ansprüche 1 bis 6, bei dem zur Durchführung des zweiten Schritts zwei separate Reaktoren verwendet werden, **dadurch gekennzeichnet, dass** der Austragsstrom aus der ersten Phase einer teilweisen Trennung der wässrigen und organischen Fraktion unterworfen wird, was die teilweise Entfernung der wässrigen Fraktion und die Rezyklierung eines Teils der organischen Fraktion, der 1 bis 10 Gew.-% des ungesättigten Nitrils repräsentiert, am Kopf des Reaktors der ersten Phase ermöglicht.

8. Verfahren nach einem der Ansprüche 1 bis 7, **dadurch gekennzeichnet, dass** ein einziger Reaktor mit H₂O₂ als einzigem Oxidationsmittel für die beiden Phasen verwendet wird, wobei das Molverhältnis von H₂O₂ zu Nitril zwischen 4/1 und 10/1 einschließlich der Grenzen liegt.

9. Verfahren nach einem der Ansprüche 1 bis 8, **dadurch gekennzeichnet, dass** im Verlauf des Reaktionsprozesses eine sequenzielle Einleitung der Katalysatoren vorgenommen wird.

10. Verwendung des Verfahrens gemäß einem der Ansprüche 1 bis 9, **dadurch gekennzeichnet, dass** sie die Herstellung von Polyamid-Monomeren, die aus ω-Aminosäuren und/oder Diaminen und/oder Disäuren, die dem Heminitril entsprechen, ausgewählt sind, betrifft und/oder dass die Verwendung den Erhalt von Polyamiden betrifft.

11. Verfahren zur Herstellung eines Polyamids, **dadurch gekennzeichnet, dass** es die Verwendung des Verfahrens gemäß einem der Ansprüche 1 bis 9 zur Herstellung eines Heminitrils ausgehend von einer ungesättigten Ausgangsfettsäure und die anschließende Hydrierung des Heminitrils zu der entsprechenden ω-Aminosäure und schließlich die Polymerisation der ω-Aminosäure zu dem Polyamid umfasst.

12. Verfahren nach Anspruch 11, **dadurch gekennzeichnet, dass** die Herstellung ausgehend von entsprechenden ungesättigten Ausgangsfettsäuren oder Ester- oder Ölderivaten der Fettsäuren, die natürlichen Ursprungs sind und aus einer erneuerbaren Quelle stammen, durchgeführt wird.

13. Verfahren nach Anspruch 11 oder 12, **dadurch gekennzeichnet, dass** es sich bei dem Polyamid um
- Polyamid 9 handelt und es sich bei der entsprechenden ungesättigten Ausgangsfettsäure um Ölsäure handelt,
- Polyamid 11 handelt und es sich bei der entsprechenden ungesättigten Ausgangsfettsäure um Vaccensäure oder Lesquerolsäure oder Gondosäure, vorzugsweise Vaccensäure oder Gondosäure und weiter bevorzugt Vaccensäure handelt.

## Claims

1. Process for synthesizing a heminitrile of formula CN-(CH₂)ₙ-COOH or of formula CN-R'-COOH, in which formulae n is between 4 and 13 limits included and R' represents an alkylene radical comprising from 4 to 13 carbon atoms and from 0 to 2 double bonds, with said synthesis being carried out from a compound of unsaturated fatty acid, including ester or glyceride, type of natural origin corresponding to the formula
(R₁-CH=CH- [(CH₂)_{q}-CH=CH]ₘ- (CH₂) ᵣ-COO-) p-G
in which formula:
R1 is H, or an alkyl radical having from 1 to 11 carbon atoms comprising, where appropriate, a hydroxyl function,
q is an index which is 0 or 1,
m and p are whole indices, m being 0, 1 or 2 and p being between 1 and 3 limits included,
if p is 1, in this case G is an H, an alkyl radical having from 1 to 11 and preferably from 2 to 11 carbon atoms, or a radical comprising two or three carbon atoms, bearing one or two hydroxyl function (s),
if p is 2, in this case G is the residue of a diol or of glycerol bearing a hydroxyl function,
if p is 3, in this case G is the residue of glycerol,
r is a whole index between 4 and 13 limits included,
with the C=C double bonds in said formula, which may be in cis or trans conformation, and with said process, comprising a first step of ammoniation of the compound of unsaturated fatty acid, ester or glyceride type, resulting in the corresponding unsaturated nitrile, which nitrile is subjected, in a second step, to an oxidative cleavage in two successive phases with the formation of intermediate compounds of vicinal diol type, using H₂O₂ as oxidizing agent, in at least one of the two phases, so as to result in said heminitrile, said process using, as unsaturated fatty acid of natural origin, oleic acid or lesquerolic acid or vaccenic acid or gondoic acid and with as the nitrile used in the second step being oleonitrile, the nitrile of lesquerolic acid or the nitrile of vaccenic acid or the nitrile of gondoic acid.

2. Process according to Claim 1, **characterized in that** the first phase of the second step, resulting in the vicinal diols, is carried out by oxidation of a double bond or double bonds using H2O2 as oxidizing agent, in the presence of an oxidation catalyst.

3. Process according to Claim 2, **characterized in that** H₂O₂ is injected into the medium in an amount representing from 1 to 4 molar equivalents, i.e. from 1 to 4 mol of the H₂O₂ per mole of unsaturated nitrile to be oxidized, more particularly in the form of an aqueous solution having an H₂O₂ content of between 30% and 70% limits included by weight, preferably between 50% and 70% limits included by weight and more preferably between 60% et 70% limits included by weight, and preferably in the presence of a catalyst consisting of tungsten derivatives, molybdenum derivatives or vanadium derivatives, more particularly chosen from: tungstic acid (H₂WO₄), the sodium salt of this acid (Na₂WO₄) combined with H₃PO₄, molybdic acid (H₂MoO₄) and its sodium salt (Na₂MoO₄), heteropoly acids such as H₃ [PMo₁₂O₄₀], H₄ [SiMo₁₂O₄₀], H₄ [SiW₁₂O₄₀], H₃[PW₁₂O₄₀] or (NH₄) ₁₀ [H₂W₁₂O₄₂] sodium metavanadate (Na₃VO₄) or ammonium metavanadate ((NH₄) ₃VO₄), and alkali metal salts thereof.

4. Process according to either of Claims 2 and 3, **characterized in that** the reaction of said second step is carried out at a temperature of between 20 and 80°C limits included and preferably between 40 and 70°C limits included and more particularly at a pressure of between 1 and 50 bar limits included, preferably at a pressure of between 1 and 20 bar limits included and more preferably at a pressure approximately equal to atmospheric pressure of 1 bar or slightly above atmospheric pressure, of between 1 and 5 bar limits not included.

5. Process according to one of Claims 2 to 4, **characterized in that** the second phase of oxidative cleavage of the diols of said second step is carried out with O₂ as oxidizing agent for cleavage of the C-C bond between the vicinal hydroxyls in the presence of a catalyst chosen from cobalt salts, such as in the form of acetate (Co(Ac)_{2·}4H₂O), chloride or sulfate or salts of Cu, Cr, Fe or Mn, and also the catalysts used during the first phase, chosen from tungstic acid (H₂WO₄) and its sodium salt Na₂WO₄ and Co/W mixtures.

6. Process according to one of Claims 2 to 4, **characterized in that** the second phase of oxidative cleavage of the vicinal diols is carried out with H₂O₂ as oxidizing agent for cleavage of the C-C bond between the vicinal hydroxyls, preferably with said H₂O₂ being injected in the form of an aqueous solution having an H₂O₂ content of between 30% and 70% limits included by weight, preferably between 50% and 70% limits included by weight and more preferably between 60% and 70% limits included by weight and with a H₂O₂/vicinal diol molar ratio of between 3/1 and 10/1 limits included.

7. Process according to one of Claims 1 to 6, using two separate reactors for implementing the second step, **characterized in that** the effluent resulting from the first phase is subjected to a partial separation of the aqueous and organic fractions, allowing the partial elimination of the aqueous fraction and the recycling, at the top of the first-phase reactor, of a part of the organic fraction, representing from 1% to 10% by weight of said unsaturated nitrile.

8. Process according to one of Claims 1 to 7, **characterized in that** a single reactor is used, with H₂O₂ as sole oxidizing agent for the two phases, the H₂O₂/nitrile molar ratio being between 4/1 and 10/1 limits included.

9. Process according to one of Claims 1 to 8, **characterized in that** a sequential injection of the catalysts is carried out through the course of the reaction process

10. Use of the process as defined according to one of Claims 1 to 9, **characterized in that** it concerns the preparation of polyamide monomers, selected from ω-amino acids and/or diamines and/or diacids equivalent to said heminitrile and/or **in that** said use concerns the obtaining of polyamides.

11. Process for producing a polyamide, **characterized in that** it comprises the use of the process as defined according to one of Claims 1 to 9 for preparing a heminitrile from a starting unsaturated fatty acid, followed by the hydrogenation of said heminitrile so as to obtain a corresponding ω-amino acid and finally the polymerization of said ω-amino acid so as to obtain said polyamide.

12. Process according to Claim 11, **characterized in that** said production is carried out from corresponding starting unsaturated fatty acids or from ester or oil derivatives of said fatty acids of natural origin and of renewable source.

13. Process according to Claim 11 or 12, **characterized in that** said polyamide is:
- polyamide 9 and said corresponding starting unsaturated fatty acid is oleic acid,
- polyamide 11 and said corresponding starting unsaturated fatty acid is vaccenic acid or lesquerolic acid or gondoic acid, preferably vaccenic acid or gondoic acid and more preferentially vaccenic acid.
